# EUROPEAN PATENT SPECIFICATION

(11) **EP 2 501 704 B1**
(45) Date of publication and mention of the grant of the patent: **18.09.2013**
(21) Application number: 10782486.4
(22) Date of filing: 12.11.2010
(51) Int. Cl.: C07D 495/20, A61K 31/438, A61P 25/00

(54) **Spiropiperidine compounds as oral-1 receptor antagagonisten**
Spiropiperidinverbindungen als ORL-1-Rezeptorantagonisten
Composés de spiropipéridine en tant qu'antagonistes du récepteur orl-1

(30) Priority: 16.11.2009 EP 09382245; 27.01.2010 US 298628 P
(43) Date of publication of application: 26.09.2012
(73) Proprietor: Eli Lilly and Company, Indianapolis, IN 46285 (US)
(72) Inventor: BENITO COLLADO, Ana Belen, Madrid (ES); DIAZ BUEZO, Nuria, Madrid (ES); JIMENEZ-AGUADO, Alma Maria, Madrid (ES); LAFUENTE BLANCO, Celia, Madrid (ES); MARTINEZ-GRAU, Maria Angeles, Madrid (ES); PEDREGAL-TERCERO, Concepcion, Madrid (ES); TOLEDO ESCRIBANO, Miguel Angel, Madrid (ES)
(74) Representative: Smith, Andrew George
(86) International application number: PCT/US2010/056449
(87) International publication number: WO 2011/060217

(56) References cited:
- WO-A1-2005/016913
- WO-A1-2005/066183
- YOSHIZUM TAKASHI ET AL: "Design, synthesis, and structure-activity relationship study of a novel class of ORL1 receptor antagonists based on N-biarylmethyl spiropiperidine", BIOORGANIC & MEDICINAL CHEMISTRY LETTERS,, vol. 18, 16 May 2008 (2008-05-16), pages 3778-3782, XP002572595, cited in the application

## Description

Orphanin FQ (OFQ)/Nociceptin is a 17 amino acid peptide that has high affinity for the ORL1 G-protein coupled receptor (GPCR). The ORL1 receptor is a Class A GPCR that is expressed primarily in the central nervous system and peripheral nervous system as well as in the gastrointestinal tract, smooth muscle, and immune system. While related structurally to opioid peptides/receptors the OFQ/Nociceptin system exhibits no significant cross reactivity to classical opioid peptides/receptors and exhibits anti-opioid activity in vivo (for example ORQ/Nociceptin has been reported to exhibit anti-nociceptive properties).

Nociceptin/orphanin FQ receptor (NOC/OFQ) antagonists, specifically antagonists of the ORL-1 receptor have demonstrated anti-depressant activity and anorectic activity in numerous studies with animal models for depression and feeding behavior. As such, ORL-1 antagonists are deemed to be useful in the treatment of depression and/or the treatment of overweight, obesity, and/or weight maintenance post treatment for overweight or obesity.

WO 2003/095427 describes certain spiropiperidinyl compounds as ORL-1 antagonists for use as analgesics.

Yoshizumi, Takashi et al. (2008), Design, synthesis, and structure-activity relationship study of a novel class of ORL-1 receptor antagonists based on N-biarylmethyl spiropiperidine, Bioorganic & Medicinal Chemistry Letters vol. 18, pg. 3778-3782, describes certain *N*-biarylmethyl-spiropiperidine compounds as selective ORL-1 antagonists.
The present invention provides a family of 4',5'-dihydrospirolpiperidine-4,7'-thieno[2,3-c]pyran] compounds with high antagonist potency for the ORL-1 receptor and high in vivo ORL-1 receptor occupancy in the CNS. Additionally, certain of the compounds have a favorable cardiotoxicology profile as determined by selectivity over the hERG channel activity, as well as high selectivity over other physiologically important receptors (e.g. mu, kappa and delta opioids, serotonin, and dopamine receptors). Further, certain of the compounds of the present invention have favorable biopharmaceutical and pharmacokinetic properties (e.g. solubility, oral exposure, and CNS permeability). Certain of the compounds of the present invention exhibit reduced oxidative metabolism resulting in favorable oral bioavailability. Certain compounds have also demonstrated through animal models that the compounds of the present invention are useful for the treatment of migraine.

The present invention provides compounds of Formula I: 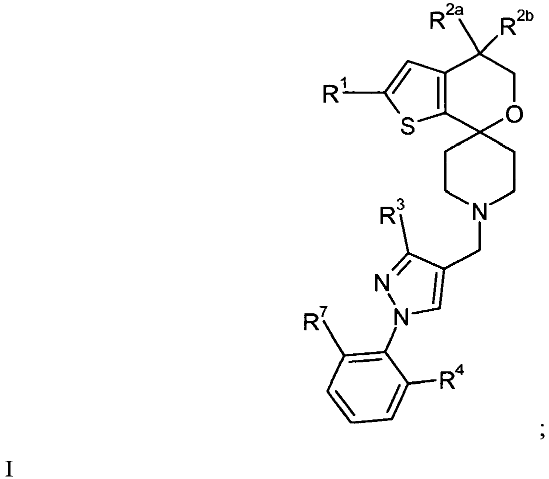 wherein
- R¹: is fluoro or chloro;
- R^{2a} and R^{2b}: are each hydrogen or are each fluoro;
- R³: is hydrogen, methyl, hydroxymethyl, or (C₁-C₃) alkoxymethyl;
- R⁴: is selected from the group consisting of fluoro, chloro, cyano, cyanomethyl, (C₁-C₃) alkyl, cyclopropyl, hydroxymethyl, methoxy, methoxymethyl, aminocarbonyloxymethyl, methylaminocarbonyloxymethyl, dimethylaminocarbonyloxymethyl, methylcarbonyl, aminocarbonyl, methylaminocarbonyl,_dimethylaminocarbonyl, NR⁵R⁶, -CH₂-NR⁵R⁶, morpholin-4-yl, morpholin-4-ylmethyl, Ar¹, -CH₂Ar¹, 3,3-difluoroazetidin-1-ylmethyl, pyrrolidin-1-ylmethyl, 1-aminocyclopropyl, 1-methylaminocyclopropyl, and 1-dimethylaminocyclopropyl;
- R⁵: is hydrogen, C₁-C₄ alkyl, cyclopropyl, hydroxyethyl, methoxyethyl, -C(O)CH₃, or -C(O)O(C₁-C₃) alkyl;
- R⁶: is hydrogen or methyl;
- R⁷: is hydrogen, fluoro, chloro, methyl, hydroxymethyl, or methoxy; and
- Ar¹: is a moiety selected from the group consisting of imidizol-1-yl, imidizol-2-yl, 2-methylimidizol-1-yl, 1-methylimidizol-2-yl, and 1,2,4-triazol-3-yl; or
a pharmaceutically acceptable salt thereof.

In another aspect of the invention there is provided a pharmaceutical composition comprising a compound of Formula I or a pharmaceutically acceptable salt thereof, in combination with a pharmaceutically acceptable carrier, diluent, or excipient. One embodiment of this aspect of the invention, the pharmaceutical composition further comprises at least one additional therapeutic ingredient, as for example an SSRI antidepressant, as for example fluoxetine. Furthermore, this aspect of the invention provides a pharmaceutical composition adapted for the treatment of depression comprising a compound of Formula I or a pharmaceutically acceptable salt thereof, in combination with one or more pharmaceutically acceptable excipients, carriers, or diluents thereof. In another embodiment of this aspect of the invention there is provided a pharmaceutical composition adapted for the treatment of overweight, obesity and/or weight maintenance, comprising a compound of Formula I or a pharmaceutically acceptable salt thereof, in combination with one or more pharmaceutically acceptable excipients, carriers, or diluents thereof. A further embodiment provides a pharmaceutical composition adapted for the treatment of migraine comprising a compound of Formula I or a pharmaceutically acceptable salt thereof, in combination with one or more pharmaceutically acceptable excipients, carriers, or diluents thereof.

The present invention also provides a method of treating depression in a mammal comprising administering to a mammal in need of such treatment an effective amount of a compound of Formula I or a pharmaceutically acceptable salt thereof. A further embodiment provides a method of treating depression in a mammal comprising administering to a mammal in need of such treatment an effective amount of a compound of Formula I, or a pharmaceutically acceptable salt thereof, and an effective amount of an SSRI antidepressant, or a pharmaceutically acceptable salt thereof, as for example fluoxetine. Other embodiments of the invention provide methods of treating overweight and/or obesity, and/or a method for weight maintenance comprising administering to a mammal in need of such treatment an effective amount of a compound of Formula I, or a pharmaceutically acceptable salt thereof. In one particular embodiment of these aspects of the invention, the mammal is a human.

This invention also provides a compound of Formula I or a pharmaceutically acceptable salt thereof for use in therapy. Within this aspect, the invention provides a compound of Formula I, or a pharmaceutically acceptable salt thereof, for use in the treatment of depression in mammals, particularly humans. The invention also provides a compound of Formula I, or a pharmaceutically acceptable salt thereof, in combination with an SSRI antidepressant, or a pharmaceutically acceptable salt thereof, as for example fluoxetine, for use in the treatment of depression in mammals, particularly humans. Further, this aspect of the invention includes any one of the following: a compound of Formula I, or a pharmaceutically acceptable salt thereof, for use in the treatment of overweight; a compound of Formula I, or a pharmaceutically acceptable salt thereof, for use in the treatment of obesity; a compound of Formula I, or a pharmaceutically acceptable salt thereof, for use in the maintenance of weight (for weight maintenance), particularly after treatment for overweight or obesity; a compound of Formula I, or a pharmaceutically acceptable salt thereof, for use in the treatment of migraine.

Another aspect of this invention provides the use of a compound of Formula I, or a pharmaceutically acceptable salt thereof, in the manufacture of a medicament for the treatment of depression. Another embodiment of the invention provides the use of a compound of Formula I, or a pharmaceutically acceptable salt thereof, in the manufacture of a medicament for the treatment of overweight, obesity, and/or the maintenance of weight. Yet another embodiment of the invention provides the use of a compound of Formula I, or a pharmaceutically acceptable salt thereof, in the manufacture of a medicament for the treatment of migraine.

Compounds of this invention are bases, and accordingly react with a number of organic and inorganic acids to form pharmaceutically acceptable salts. Pharmaceutically acceptable salts of each of the compounds of the present invention are contemplated within the scope of the present application. The term "pharmaceutically acceptable salt" as used herein, refers to any salt of a compound of Formula I that is substantially non-toxic to living organisms. Such salts include those listed in Journal of Pharmaceutical Science, 66, 2-19 (1977), which are known to the skilled artisan.

Abbreviations used herein are defined as follows:
"BSA" means bovine serum albumin.
"mCPP" means *meta*-chlorophenylpiperazine, a non-selective serotonin receptor agonist.
"EDTA" means ethylene diamine tetraacetic acid.
"EGTA" means ethylene glycol tetraacetic acid.
"GTP" means guanosine triphosphate.
"HEPES" means 4-(2-hydroxyethyl)-1-piperazineethanesulfonic acid.
"HPLC" means high-pressure liquid chromatography.
"IC₅₀" means the concentration at which 50% of the maximum inhibition is achieved.
"LC/MS" means liquid chromatography followed by mass spectroscopy.
"LC/MS/MS" means liquid chromatography followed by mass spectroscopy, followed by a second ionizing mass spectroscopy.
"mFST" means mouse forced swim test; an animal model for antidepressant activity.
"MS" means mass spectroscopy.
"MS (ES+)" means mass spectroscopy using electrospray ionization.
"NMR" means nuclear magnetic resonance.
"RO Tracer" means 2-[(2-Fluorophenyl)methyl]-3-(2-fluorospiro[4,5-dihydrothieno[2,3-c]pyran-7,4'-piperidine]-1'-yl)-*N,N-*dimethyl-propanamide.
"RO" means receptor occupancy.
"SCX column" means strong cation exchange column.
"SNAr" means nucleophilic aromatic substitution.
"*t*Bu" means a *tertiary*-butyl moiety.
"TLC" means thin layer chromatography.
"XRD" means X-Ray Diffraction.

Preferred compounds of the present invention are compounds wherein:
1) R¹ is chloro;
2) R^{2a} and R^{2b} are each fluoro;
3) R¹ is chloro and R^{2a} and R^{2b} are each fluoro;
4) R¹ is fluoro and R^{2a} and R^{2b} are each hydrogen;
5) R³ is hydrogen, methyl, hydroxymethyl, or methoxymethyl;
6) R³ is methyl;
7) R³ is hydroxymethyl;
8) R¹ is chloro, R^{2a} and R^{2b} are each fluoro, and R³ is methyl;
9) R¹ is chloro, R^{2a} and R^{2b} are each fluoro, and R³ is hydroxymethyl;
10) R⁷ is hydrogen, fluoro, or chloro;
11) R⁷ is fluoro;
12) R¹ is chloro, R^{2a} and R^{2b} are each fluoro, and R⁷ is fluoro;
13) R¹ is chloro, R^{2a} and R^{2b} are each fluoro, R³ is methyl, and R⁷ is fluoro;
14) R¹ is chloro, R^{2a} and R^{2b} are each fluoro, R³ is hydroxymethyl, and R⁷ is fluoro;
15) R⁴ is fluoro, hydroxymethyl, methoxymethyl, methylcarbonyl or 2-methylimidazol-1-yl;
16) R⁴ is fluoro;
17) R⁴ is hydroxymethyl;
18) R⁴ is methoxymethyl;
19) R⁴ is methylcarbonyl;
20) R⁴ is 2-methylimidazol-1-yl;
21) any one of preferred embodiments 1) through 14) wherein R⁴ is fluoro;
22) any one of preferred embodiments 1) through 14) wherein R⁴ is hydroxymethyl;
23) any one of preferred embodiments 1) through 14) wherein R⁴ is methoxymethyl;
24) any one of preferred embodiments 1) through 14) wherein R⁴ is methylcarbonyl;
25) any one of preferred embodiments 1) through 14) wherein R⁴ is 2-methylimidazol-1-yl

Certain preferred compounds of the present invention are any one of
2-chloro-1'-[[1-(2,6-difluorophenyl)-3-methyl-pyrazol-4-yl]methyl]-4,4-difluoro-spiro[5H-thieno[2,3-c]pyran-7,4'-piperidine],
1-(2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-fluorophenyl)ethanone,
2-chloro-4,4-difluoro-1'-[[1-[2-fluoro-6-(2-methylimidazol-1-yl)phenyl]-3-methyl-pyrazol-4-yl]methyl]spiro[5H-thieno[2,3-c]pyran-7,4'-piperidine], and
[4-[(2-chloro-4,4-difluoro-spiro[5H-thieno[2,3-c]pyran-7,4'-piperidine]-1'-yl)methyl]-1-(2,6-difluorophenyl)pyrazol-3-yl]methanol,
or a pharmaceutically acceptable salt thereof, as exemplified in examples 2, 40, 47, and 50.

In another preferred embodiment, it has been found that compounds wherein R^{2a} and R^{2b} are each fluoro, the compounds have a more favorable pharmacokinetic profile, being more stable to oxidative metabolism. This has the general effect of improving the oral bioavailability of the compounds.

### General Chemistry

The compounds of the present invention can be prepared according to the following synthetic schemes by methods well known and appreciated in the art. Suitable reaction conditions for the steps of these schemes are well known in the art and appropriate substitutions of solvents and co-reagents are within the skill of the art. Likewise, it will be appreciated by those skilled in the art that synthetic intermediates may be isolated and/or purified by various well known techniques as needed or desired, and that frequently, it will be possible to use various intermediates directly in subsequent synthetic steps with little or no purification. Furthermore, the skilled artisan will appreciate that in some circumstances, the order in which moieties are introduced is not critical. The particular order of steps required to produce the compounds of Formula I is dependent upon the particular compound being synthesized, the starting compound, and the relative lability of the substituted moieties, as is well appreciated by the skilled chemist. All substituents, unless otherwise indicated, are as previously defined, and all reagents are well known and appreciated in the art.

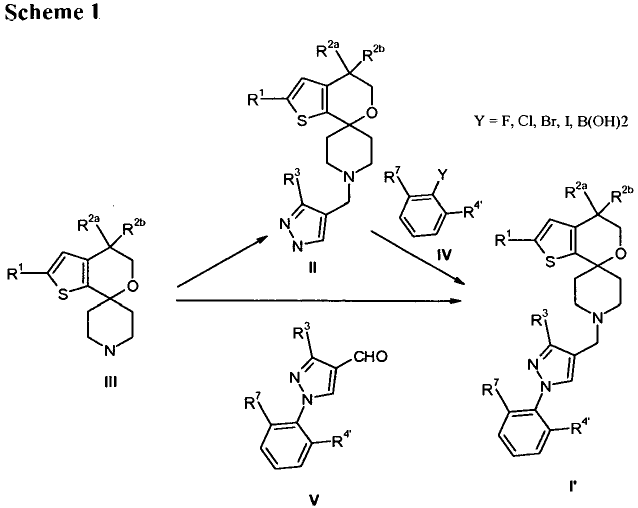

By using reductive amination reaction conditions, compound III is reacted with an appropriately substituted pyrazole carbaldehyde and a reducing reagent such as sodium triacetoxyborohydride, in a suitable solvent such as tetrohydrofuran at ambient temperature to provide compound II. Under appropriate coupling conditions, compound II is coupled with compound IV, wherein substituent Y is chloro, bromo, iodo, or boronic acid, with a suitable catalyst such as copper iodide, a proper base such as potassium carbonate, in an appropriate solvent such as toluene at elevated temperature to give compound I', wherein R^{4'} is equal to R⁴ or a precursor of R⁴. The compound of formula III can also react with an appropriately substituted aldehyde compound V under reductive amination conditions described above to give the desired compound of formula I'. When R^{4'} is a precursor to R⁴, it is then converted to R⁴ by known methods.

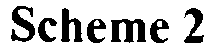

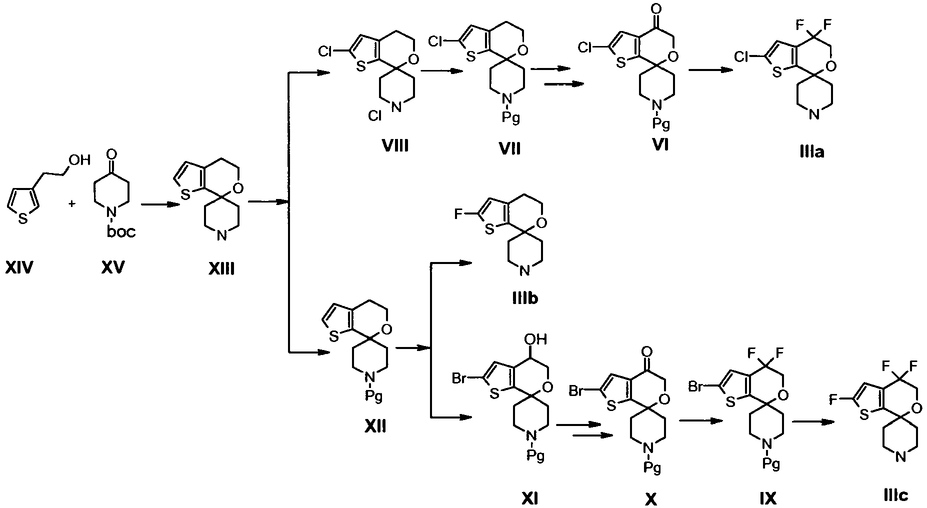

Compounds IIIa, IIIb, and IIIc can be made as illustrated in Scheme 2. Compounds XIV and XV are reacted in an appropriate solvent such as dichloromethane in the presence of a suitable acid such as trifluroacetic acid. The resultant trifluoroacetate is basified with aqueous sodium hydroxide solution to give compound XIII as a free base. Compound XIII in a suitable solvent such as methyl *t-*butyl ether is treated with a solution of sulfuryl chloride in acetic acid at ambient temperature to give compound VIII as a hydrochloride salt. Compound VIII is then protected with a nitrogen protecting group under conditions well known to the skilled artisan to give compound VII. (For example, see: Greene and Wuts, Protective Groups in Organic Synthesis, Third Edition, Chapters 2 and 7, John Wiley and Sons Inc., (1999)). Typically, the protecting group is a Boc (tert-Butyloxycarbonyl) group. Compound VII is reacted with *N*-bromosuccinimide in an appropriate solvent such as chlorobenzene with irradiation of a light source to give a bromide compound, which is then treated with a base solution such as aqueous sodium bicarbonate to afford a hydroxyl compound. With or without isolation, the hydroxyl compound can be further oxidized under suitable oxidation conditions such as in potassium bromide, tetramethylpiperidine-*N*-oxide, and aqueous sodium hypochloride solution to provide the desired ketone compound VI. Compound VI is then reacted with (bis(2-methylethyllamino)sulfur trifluoride in an appropriate solvent such as tetrahydrofuran at elevated temperature, the product obtained is de-protected to provide compound IIIa.

Compound of formula XIII is protected to give compound XII with a similar method to that used to make compound VII. Compound XII in an appropriate solvent such as tetrahydrofuran at lowered temperature is treated with a suitable base such as lithium tetramethylpiperidide, followed by addition of *N*-fluorobenzenesulfonimide to afford a fluoride compound, which is de-protected with aqueous HCl and basified with aqueous NaOH solution to provide compound IIIb.

By the same method used for making compound VI above, compound IX can be obtained from compound XII through three-step synthesis, such as bromination, hydroxylation, and oxidation. Each intermediate can be isolated as pure compound for further reaction or reacted without isolation as described in the synthesis of compound VI. Compound IX is then treated with a suitable halogen-metal exchange reagent such as butyl lithium in proper solvent such as tetrahydrofuran under lowered temperature, followed by a fluorinating reagent such as *N*-fluoro-benzenesulfonimide to afford the desired fluorinated product, which is then de-protected appropriately to give the desired compound IIIc.

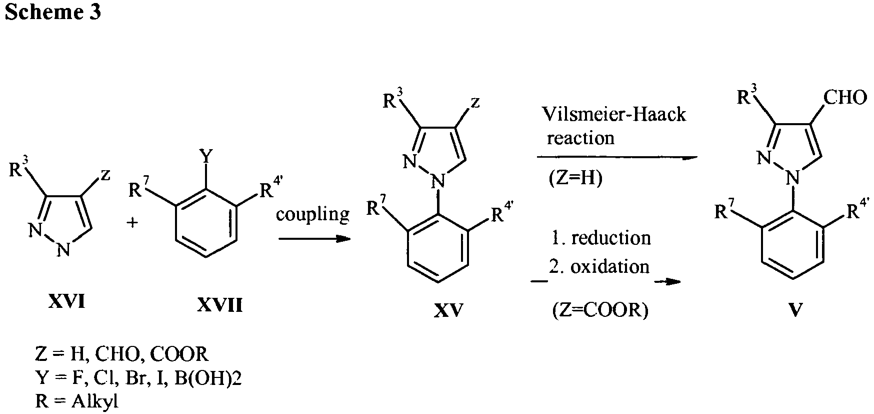

Compound of formula V can be made as illustrated in Scheme 3. Compound XVI is reacted with compound XVII to afford compound of formula XV under coupling conditions described above for the conversion of compound II to compound I'. When Y is F or Cl, nucleophilic aromatic substitution (SNAr) is an alternative method to make compound of formula XV. More specifically, compound XVI can react with compound XVII in an appropriate solvent such as dimethylformamide with a suitable base such as potassium carbonate at elevated temperature to provide compound XV. When Z is hydrogen, it can be converted to an aldehyde with the Vilsmeier-Haack reaction. When Z is an ester group, it can be reduced to an alcohol first with an appropriate reducing reagent such as lithium aluminum hydride in an appropriate solvent such as tetrahydrofuran. The alcohol is then oxidized to an aldehyde with an appropriate oxidation reagent such as manganese (IV) oxide in a solvent such as dichloromethane.

When R^{4'} is the precursor of R⁴, the transformation of R^{4'} to R⁴ will include but not limited to reactions such as reductive amination to provide an desired new amine; reduction of an ester, ketone, or aldehyde to an alcohol, which can be further converted to an alkoxy compound or a carbamate; reduction of a nitrile to an amide or an amine; the transformation of an ester to a heterocycle such as oxadiazol under proper condition. (For more examples, see: Richard C. Larock, Comprehensive Organic Transformations, Second Edition, Chapters 2 and 7, John Wiley and Sons Inc., (1999)).

The following Preparations and Examples are illustrative of methods useful for the synthesis of the compounds of the present invention. The names for many of the compounds illustrated in the preparations and examples are provided from structures drawn with 'Symyx Draw 3.1' or 'Autonom 2000 Name'.

### Preparation 1: 4',5'-Dihydrospiro[piperidine-4,7'-thieno[2,3-c]pyran]

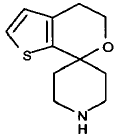

3-Thiopheneethanol (123.03 mL, 1.11 mol) is added to a solution of *N*-*tert-*butoxycarbonyl-4-piperidone (185 g, 928.48 mmol) in dichloromethane (1300 mL) and stirred at room temperature. Then trifluoroacetic acid (280.82 mL, 3.71 mole) is added dropwise (5 min) while cooling with ice/water bath (internal temperature = 14°C-30°C, caution: CO₂ evolution). The reaction mixture is gradually warmed to ambient temperature and stirred at that temperature. After 20 hr., the solvent is evaporated and a beige solid crystallized upon cooling *in vacuo.* The solid is slurried in methyl t-butyl ether(200 mL), filtered, washed with methyl t-butyl ether (2 x 1000 mL) and dried under vacuum to afford 4',5'-dihydrospiro[piperidine-4,7'-thieno[2,3-c]pyran]-1-ium trifluoroacetate as a white solid in 95% yield. MS = (m/z): 210 (M+1). 10 M sodium hydroxide (220.36 mL, 2.20 mol) is added to a stirred suspension of 4',5'-dihydrospiro[piperidine-4,7'-thieno[2,3-c]pyran]-1-ium trifluoroacetate (285 g, 881.44 mmol) in dichloromethane (1 L) with cooling (ice/water bath) and the resulting mixture is stirred till a biphasic mixture is obtained. The phases are separated and aqueous layer is extracted with dichloromethane (2x200 mL). Combined organics are concentrated under vacuum to obtain a dense oil which is triturated with water to obtain a light yellow precipitate. It is filtered, washed with water (300 mL) and hexane (200 mL) and dried under vacuum at 35°C for 20 hr. to yield title compound as a light yellow solid in 86% yield. MS (m/z): 210 (M+1).

### Preparation 2: tert-Butyl spiro[4,5-dihydrothieno[2,3-c]pyran-7,4'-piperidine]-1'-carboxylate

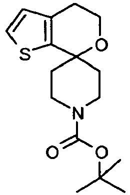

Spiro[4,5-dihydrothieno[2,3-c]pyran-7,4'-piperidine] (60 g, 286.6 mmol) in 2-methyltetrahydrofuran (600 mL) is stirred at 22°C for 10 min. Then, tert-butoxycarbonyl tert-butyl carbonate (65.6 g, 301 mmol) in 2-methyltetrahydrofuran (300 mL) is added dropwise. After 12 hours, aqueous solution of sodium chloride (250 mL) is added and the organic layer separated. Then aqueous layer is washed twice with 2-methyltetrahydrofuran (2 x 50 mL) and the organic layers are combined and washed with brine, dried over sodium sulfate, filtered, and concentrated under reduced pressure to give the title compound in 99% yield. MS (m/z): 310 (M+1).

### Preparation 3: 2-Fluorospiro[4,5-dihydrothieno[2,2-c]pyran-7,4'-piperidine]

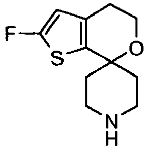

### 1. tert-Butyl 2-fluorospiro[4,5-dihydrothieno[2,3-c]pyran-7,4'-piperidinel-1'-carboxylate

2,2,6,6-Tetramethylpiperidine (18.7 mL, 110.5 mmol) is added over tetrahydrofuran (200 mL), and solution is cooled under nitrogen at -78°C. 2.5 M solution of butyl lithium in hexane (37.2 mL, 93 mmol) is added and mixture is stirred for 30 min at -78°C. Over the fresh lithium 2,2,6,6-tetramethylpiperidine solution is added a solution of tert-butyl spiro[4,5-dihydrothieno[2,3-c]pyran-7,4'-piperidine]-1'-carboxylate (20 g, 58.2 mmol) in tetrahydrofuran (90 mL) keeping temperature below -70°C. After 20 min a solution of *N*-fluorobenzenesulfonimide (30.26 g, 93.07 mmol) in tetrahydrofuran (200 mL) previously cooled under nitrogen at -20°C is added via cannula. After 1 hr. stirring, water (20 mL) and aqueous solution of ammonium chloride (50 mL) are added. Then, organic layer is separated and the aqueous is washed twice with methyl t-butyl ether (2 x 25 mL). Organics are combined and solvent is evaporated under reduced pressure. Crude material is purified by normal phase HPLC using hexane/ methyl t-butyl ether as solvents to give tert-butyl 2-fluorospiro[4,5-dihydrothieno[2,3-c]pyran-7,4'-piperidine]-1'-carboxylate in 50% yield. MS (m/z): 328 (M+1).

### 2. 2-Fluorospiro[4,5-dihydrothieno[2,2-c]pyran-7,4'-piperidine]

37% Hydrochloric acid (11.75 mL, 125.22 mmol) is added to a solution of tert-butyl 2'-fluoro-4',5'-dihydro-1 H-spiro[piperidine-4,7'-thieno[2,3-c]pyran]-1-carboxylate (8.2 g, 25.04 mmol) in isopropyl alcohol (57.4 mL) at 45 °C. The resulting solution is stirred at 45°C for 6.5 hr. The solvent is concentrated to a yellow suspension. Water (50 mL) is added and the mixture is basified with 5N aqueous solution of sodium hydroxide. The aqueous phase is extracted with ethyl acetate (3 x 100 mL) and the combined organic extracts are washed with brine (50 mL), dried over sodium sulfate, filtered and concentrated under reduced pressure to give the title compound in 96% yield. MS (m/z): 228 (M+1).

### Preparation 4: 2'-Chloro-4',5'-dihydrospiro[piperidine-4,7'-thieno[2,3-c]pyran] hydrochloride

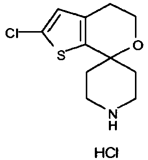

A solution of 4',5'-dihydrospiro[piperidine-4,7'-thieno[2,3-c]pyran] (50 g, 238.88 mmol) in a mixture of acetic acid (400 mL) and methyl t-butyl ether(40 mL) is cooled to 15°C. Then, a solution of sulfuryl chloride (21.20 mL, 262.77 mmol) in acetic acid (100 mL) is added dropwise in 40 min at that temperature (internal temperature = 15°C-22°C) and the mixture is stirred at room temperature for 20 hr. Then, a solution of sulfuryl chloride (11.56 mL, 143.33 mmol) in acetic acid (50 mL) is added dropwise at rt. Reaction mixture is stirred at room temperature for 30 min and then it is added dropwise (30 min) over methyl t-butyl ether (1 L) cooling with ice/water bath with stirring. A white suspension is formed and the solid is filtered. To obtain a second crop of material, the filtrate is concentrated (refilling with methyl t-butyl ether via rotavap). Resulting solid is suspended in methyl t-butyl ether (300 mL), suspension is stirred at reflux (bath: 100 °C) and methanol (30 mL) is added till a cloudy suspension is formed. Then, the suspension is cooled to room temperature overnight. The suspension is further cooled in a ice/water bath and filtered. Solid is washed with methyl t-butyl ether (50 mL) and combined with first crop to give the title compound in a 60% yield. MS (m/z): 244 (M+1).

### Preparation 5: 2'-Chloro-4',4'-difluoro-4',5'-dihydrospiro[piperidine-4,7'-thieno[2,3-c]pyran]

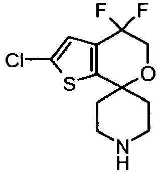

### 1. tert-Butyl 2-chlorospiro[4,5-dihydrothieno[2,3-c]pyran-7,4'-piperidine]-1'-carboxylate

To a suspension of 2-chlorospiro[4,5-dihydrothieno[2,2-c]pyran-7,4'-piperidine] hydrochloric salt (140 g, 0.49 moles) in dichloromethane (1.12 L) is added triethylamine (67.25 mL, 1.05 mole), 4-pyridinamine, *N,N*-dimethyl- (3.05 g, 0.025 mole) and di-t-butyldicarbonate (111.22 g, 0.509 mole) in portions and the resulting mixture is stirred at room temperature overnight. The reaction is washed with 1N HCl (2 x) and water. The organic phase is dried over magnesium sulfate, filtered and concentrated under reduced pressure to afford *tert*-butyl 2-chlorospiro[4,5-dihydrothieno[2,3-c]pyran-7,4'-piperidine]-1'-carboxylate in 53% yield. MS (m/z): 244 (M+1-Boc).

### 2. tert-Butyl 4'-oxo-2'-chloro-4',5'-dihydrospiro[piperidine-4,7'-thieno[2,3-c]pyran]-1-carboxylate

In a 5L jacketed reactor, *N*-bromosuccinimide (115.02 g, 639.77 mmol) is added to a solution of *tert*-butyl 2-chlorospiro[4,5-dihydrothieno[2,3-c]pyran-7,4'-piperidine]-1'-carboxylate (200 g, 581.61 mmol) in chlorobenzene (1.60 L) at rt. The resulting suspension is irradiated with 3 X 100 w bulb lamps situated almost in contact with the external reactor wall and the reactor temperature is set to 45°C. After 4 hr., *N*-bromosuccinimide (26.14 g, 145.40 mmol) is added and the temperature is maintained at 40°C for 15 hr. Reaction mixture is cooled to 0°C and methyl t-butyl ether (500 mL) is added. Solid is filtered and the solution is concentrated to about 1000 mL solution in chlorobenzene. Then, methyl t-butyl ether (1000 mL) is added, solids are filtered and filtrate is concentrated to afford 600 mL of chlorobenzene solution. Dimethyl sulfoxide (806.47 mL, 11.35 mol) is added and sodium bicarbonate (95.38 g, 1.14 mol) are added at room temperature. After stirring 24 hr. at room temperature, water/ice (1000 mL) is added and the phases are separated. Organic phase is washed with water (2 x 1 L) and concentrated to afford a solution in chlorobenzene. Then, dichloromethane (1.2 L) is added and the mixture is cooled to 5°C (ice/water bath). Potassium bromide (20.27 g, 170.31 mmol) and 2,2,6,6-tetramethylpiperidine-N-oxide (4.43 g, 28.38 mmol) are added. Then, a solution of sodium hypochlorite 6% in water (644.40 mL, 567.68 mmol) adjusted at pH = 9 with sodium bicarbonate (s) is added to the reaction mixture at 5°C and the resulting mixture is stirred 1 hr. at 5°C. Room temperature water (1 L) is added and the phases are separated. Organic phase is washed with water (2 x 0.5 L) and was cooled with ice/water bath. Then, Potassium Bromide (2.03 g, 17.03 mmol), 2,2,6,6-tetramethylpiperidine-*N*-oxide (0.05 g, 0.32 mmoles) and a solution of 6% sodium hypochlorite in water (128.88 mL, 113.54 mmol) adjusted at pH = 9 with solid sodium bicarbonate are added to the reaction mixture at 5°C and the resulting mixture is stirred 1 hr. from 5°C to room temperature. Then, water (1 L) is added and the phases are separated. Organic phase is washed with water (2 x 1 L) dried and concentrated to afford a dark brown solid.

Solid is triturated with hexane (500 mL), methyl t-butyl ether/hexane 5% (250 mL) and methyl t-butyl ether/hexane 10% (250 mL) to obtain tert-butyl 4'-oxo-2'-chloro-4',5'-dihydrospiro[piperidine-4,7'-thieno[2,3-c]pyran]-1-carboxylate as a light brown solid in a 66% yield. MS (m/z): 258 (M+1-Boc).

### 3. tert-Butyl 2'-chloro-4',4'-difluoro-4',5'-dihydrospirotpiperidine4,7'-thieno[2,3-c]pyran]-1-carboxylate

In a 500 mL PFA flask charged with tetrahydrofuran (81 mL) is added (bis(2-methoxyethyl)amino)sulfur trifluoride (183.62 g, 829.94 mmol) and tert-butyl 4'-oxo-2'-chloro-4',5'-dihydrospiro[piperidine-4,7'-thieno[2,3-c]pyran]-1-carboxylate (135 g, 377.24 mmol). The resulting suspension is stirred at 70°C for 24h. Then, it is cooled to room temperature and slowly poured over a mixture of ice and saturated aqueous solution of sodium bicarbonate (4L) with stirring (gas evolution). Methyl t-butyl ether is used to transfer the remaining material from flasks. After gas evolution ceased, solid sodium bicarbonate is added with stirring until pH 8 was reached. The resulting mixture is extracted with methyl t-butyl ether (3 x 500 mL) until no product was detected by TLC in the aqueous phase. Combined organics are washed with water (3 x 500 mL) and brine (500 mL), dried over sodium sulfate and concentrated to afford a dark thick oil (250 g). Crude material is dissolved in dichloromethane and filtered through a silica gel plug eluting with methyl t-butyl ether/hexane 10% (6 L) and methyl t-butyl ether/hexane (4 L). Fractions are collected till no product was detected by TLC (20% methyl t-butyl ether/hexane, UV, Rf = 0.5). Filtrate is concentrated to obtain a light brown solid which is dried under vacuum at 40°C till constant weight to afford 70% yield of tert-butyl 2'-chloro-4',4'-difluoro-4',5'-dihydrospiro[piperidine-4,7'-thieno[2,3-c]pyran]-1-carboxylate. MS (m/z): 324 (M+1-tBu).

### 4. 2'-Chloro-4',4'-difluoro-4',5'-dihydrospiro[piperidine-4,7'-thieno[2,3-c]pyran]

37% Hydrochloric acid (74.12 mL, 789.78 mmol) is added to a solution of *tert*-butyl 2'-chloro-4',4'-difluoro-4',5'-dihydrospiro[piperidine-4,7'-thieno[2,3-c]pyran]-1-carboxylate (60 g, 157.96 mmol) in isopropyl alcohol (420 mL) at 45°C. The resulting solution is stirred at 45°C for 15 hr. Then, the mixture is concentrated to 1/4 volume to afford a white suspension. Water (100 mL) is added and the suspension is basified with 6N aqueous solution of sodium hydroxide to obtain a two-layer mixture that is extracted with methyl t-butyl ether (3 x 100 mL). Combined organics are washed with brine (50 mL), dried over sodium sulfate and concentrated to afford a light brown solid which is dried under vacuum till constant weigh to yield 97% of the title compound. MS (m/z): 280 (M+1).

### Preparation 6: 2,4,4-Trifluorospiro[5H-thieno[2,3-c]pyran-7,4'-piperidine]

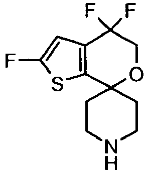

### 1. tert-Butyl 2-bromo-4-hydroxy-spiro[4,5-dihydrothieno[2,3-c]pyran-7,4'-piperidine]-1'-carboxylate

*N*-bromosuccinimide (2.2 equiv) is added to a solution of tert-butyl spiro[4,5-dihydrothieno[2,3-c]pyran-7,4'-piperidine]-1'-carboxylate (13.5 g) in chlorobenzene (108 mL). The resulting suspension is irradiated with 260 w bulb lamp overnight. More *N*-bromosuccinimide (1.7 g) is added to the mixture and the mixture is irradiated with 260 w bulb lamp for 3 hours. The solvent is eliminated under reduced pressure giving a residue that is dissolved in acetone (650 mL) and a solution of silver nitrate (8.8g) in water (650 mL) is added. The mixture is stirred at room temperature in darkness overnight. The mixture is filtered off and the acetone is evaporated. Ethyl acetate is added and the organic layer is washed with saturated aqueous solution of sodium bicarbonate and brine. The organic layer is dried over sodium sulfate, filtered and the solvent is evaporated under reduced pressure. The residue is purified by normal phase Isco chromatography (eluent: hexane/ethyl acetate 15-60%) to afford *tert*-butyl-2-bromo-4-hydroxy-spiro[4,5-dihydrothieno[2,3-c]pyran-7,4'-piperidine]-1'-carboxylate in a 38% yield. MS (m/z): 426/428 (M+23/M+2+23)

### 2. tert-Butyl 2'-bromo-4'-oxo-spiro[piperidine-4,7'-thieno[2,3-c]pyran]-1-carboxylate

Potassium bromide (535.67 mg, 4.50 mmol) is added to a solution of tert-butyl 2-bromo-4-hydroxy-spiro[4,5-dihydrothieno[2,3-c]pyran-7,4'-cyclohexane]-1'-carboxylate (7.28g) and 2,2,6,6-tetramethylpiperidine-*N*-oxide (281.33 mg, 1.80 mmol,) in dichloromethane (70 mL) at 0°C. In another vessel sodium bicarbonate is added to 10% in water sodium hypochlorite (22.34 mL, 36.01 mmol) until pH 9. This sodium hypochlorite-sodium bicarbonate solution is added dropwise at 0°C and the resulting dark suspension is stirred at 0°C for 15 min. dichloromethane (20 mL) and water (20 mL) are added and the phases are separated. The organic phase is washed with water (20 mL) and dried over sodium sulfate. The solvent is eliminated under reduced pressure to afford tert-butyl 2'-bromo-4'-oxo-spiro[piperidine-4,7'-thieno[2,3-c]pyran]-1-carboxylate in a 99% yield. MS (m/z): 346/348 (M- t-Bu)

### 3. tert-Butyl 2-bromo-4,4-difluoro-spiro[5H-thieno[2,3-c]pyran-7,4'-piperidine]-1'-carboxylate

In a 100 mL perfluoroalcoxy-flask flask is added (bis(2-methoxyethyl)amino)sulfur trifluoride (5.16 mL, 27.96 mmol) to dry tetrahydrofuran (3.5 mL). Then tert-butyl 2'-bromo-4'-oxo-spiro[piperidine-4,7'-thieno[2,3-c]pyran]-1-carboxylate (4.5 g, 11.19 mmol) is added. The solution is stirred at 70 °C overnight. After that time, methyl t-butyl ether is added (30 mL), and the reaction mixture is cautiously poured over sodium bicarbonate (saturated aqueous solution) cooled in an ice bath. CO2 evolution is seen and sodium bicarbonate (saturated aqueous solution) is added until pH 8. The mixture is extracted with methyl t-butyl ether. Organic layer is decanted, washed with brine (2 x), dried over magnesium sulfate and the solvent evaporated under reduced pressure. The crude is purified by normal phase Isco chromatography eluting with methyl t-butyl ether/hexane to yield 3.2 g of *tert*-butyl 2-bromo-4,4-difluoro-spiro[5H-thieno[2,3-c]pyran-7,4'-piperidine]-1'-carboxylate. MS (m/z): 368 (M-55).

### 4. tert-Butyl 2,4,4-trifluorospiro[5H-thieno[2,3-c]pyran-7,4'-piperidine]-1'-carboxylate

2.5 M butyl lithium in hexane (47 mL) is added over a solution of *tert-butyl* 2-bromo-4,4-difluoro-spiro[5H-thieno[2,3-c]pyran-7,4'-piperidine]-1'-carboxylate (1.99 g, 4.69 mmol) in tetrahydrofuran (50 mL) under nitrogen and at -78°C. The mixture is stirred at -78°C for 1 hour and solid *N*-fluoro-benzenesulfonimide (3.69 g, 11.73 mmol) is added. The mixture is allowed to warm to room temperature and stirred at room temperature overnight. Saturated aqueous solution of ammonium chloride is added and the organic phase is extracted with ethyl acetate, dried over sodium sulfate and the solvent eliminated under reduced pressure. The crude material is purified by normal phase Isco chromatography (hexane/ethyl acetate 3-12%) to yield 1.3 g of *tert*-butyl 2,4,4-trifluorospiro[5H-thieno[2,3-c]pyran-7,4'-piperidine]-1'-carboxylate that is further purified by HPLC to obtain 0.818 g of that compound. MS (m/z): 308 (M-t-Bu).

### 5. 2,4,4-Trifluorospiro[5H-thieno[2,3-c]pyran-7,4'-piperidine]

*t*-Butyl 2,4,4-trifluorospiro[5H-thieno[2,3-c]pyran-7,4'-piperidine]-1'-carboxylate (2.00g, 5.50 mmol) is dissolved in hydrochloric acid (4N in dioxane) (10 mL, 40 mmol). The mixture is stirred at room temperature for 1 hr. and then passed through a 50 g SCX cartridge to yield 1.3 g of the title compound after evaporation of 2 N ammonia in methanol fraction. MS (m/z): 264 (M+1).

### Preparation 7: 2-Chloro-4,4-difluoro-1'-[(3-methyl-1H-pyrazol-4-yl)methyl]spiro[5H-thieno[2,3-c]pyran-7,4'-piperidine]

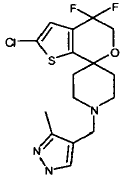

To a solution of 2-chloro-4,4-difluoro-spiro[5H-thieno[2,3-c]pyran-7,4'-piperidine] (105 g, 375 mmol) in tetrahydrofuran (1.58 L) is added 3-methyl-1H-pyrazole-4-carbaldehyde (43.40 g , 394.12 mmol) and the mixture is stirred at room temperature for 1 hr. Then, powdered sodium triacetoxyborohydride (95.46 g, 450.42 mmol) is added in 3 portions. The mixture is stirred at room temperature for 15 hr. Then, the reaction mixture is poured over an ice-sodium bicarbonate saturated aqueous solution (400 mL). Phases are separated. Aqueous phase is extracted with ethyl acetate (100 mL). Combined organic layers are washed with 50% brine and a solid precipitates in the organic phase. Organic phase is concentrated to give 170 g of the title compound. MS (m/z): 374 (M+1).

The compounds of Preparation 8-11 are prepared essentially as described in Preparation 7 using 1,2-dichloroethane as solvent..

| Prep. No. | Chemical name | Structure | Yield (%) | Physical data: MS (m/z) |
|---|---|---|---|---|
| 8 | 2-Fluoro-1'-[(3-methyl-1H-pyrazol-4-yl)methyl]spiro[4,5-dihydrothieno[2,3-c]pyran-7,4'-piperidine] | 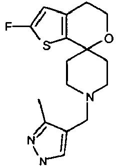 | 93 | 322 (M+1) |
| 9 | 2-Chloro-1'-[(3-methyl-1H-pyrazol-4-yl)methyl]spiro[4,5-dihydrothieno[2,3-c]pyran-7,4'-piperidine] | 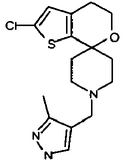 | 99 | 338 (M+1) |
| 10 | 2,4,4-Trifluoro-1'-[(3-methyl-1H-pyrazol-4-yl)methyl]spiro[5H-thieno[2,3-c]pyran-7,4'-piperidine] | 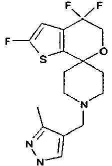 | 99 | 358 (M+1) |
| 11 | 2-Chloro-1'-(1H-pyrazol-4-ylmethyl)spiro[4,5-dihydrothieno[2,3-c]pyran-7,4'-piperidine] | 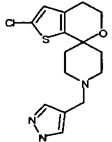 | 99 | 324 (M+1) |

### Preparation 12: 1-(2,6-Difluorophenyl)-3-methyl-pyrazole-4-carbaldehyde

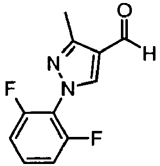

### 1. 1-(2,6-Difluoro-phenyl)-3-methyl-1H-pyrazole

To a screw-cap test tube are added copper(I) iodide (1.86 g, 9.74 mmoles), 3-methyl-1-H-pyrazole (3.92 mL, 48.72 mmoles) and potassium carbonate (14.28 g, 102.31 mmoles). Then, 2-bromo-1,3-difluoro-benzene (13.75 mL, 121.80 mmoles) and *trans*-N,N'-dimethylcyclohexane-1,2-diamine (3.07 mL, 19.49 mmoles) are added and the mixture is stirred at 115°C for 24 hr. Reaction is cooled down, water (50 mL) is added and the mixture is extracted with dichloromethane (3 x 20 mL). Organics are dried over sodium sulfate and concentrated to afford a dark oil. The residue is purified by silica gel chromatography using as eluent dichloromethane/hexane (from 50 to 100% of dichloromethane) to afford 2.5 g of 1-(2,6-difluoro-phenyl)-3-methyl-1H-pyrazole. MS (m/z): 195 (M+1).

### 2. 1-(2,6-Difluorophenyl)-3-methyl-pyrazole-4-carbaldehyde

Phosphoryl chloride (9.19 mL, 98.88 mmol) is added dropwise to dimethylformamide (7.65 mL, 98.88 mmol) at 0°C and the mixture is stirred 10 min at that temperature. Then 1-(2,6-difluoro-phenyl)-3-methyl-1H-pyrazole (4.80 g, 24.72 mmol) is added to the resulting suspension and the reaction mixture is stirred at 85°C for 20 hr. Reaction mixture is cooled to room temperature and ice and water (20 mL) are added with no exotherm. The mixture is poured over saturated aqueous solution of sodium bicarbonate (30 mL) and basified with 2 N sodium hydroxide. The mixture is extracted with methyl t-butyl ether(2 x 30 mL) and the organic layer is washed with brine (2 x 30 mL), dried over sodium sulfate and the solvent evaporated under reduced pressure to give 5.3 g of the title compound. MS (m/z): 223 (M+1).

### Preparation 13: Ethyl 3-methyl-1H-pyrazole-4-carboxylate

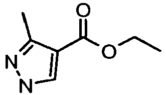

Sulfuric acid (18 mL, 337.69 mmol) is added to a suspension of 1H-pyrazole-4-carboxylic acid, 3-methyl- (10 g, 79.29 mmol) in ethanol (90 mL) and the mixture is stirred at 85°C for 20 hr. After that time, solvent is partially removed. Residue is basified with 1M sodium hydroxide solution to pH 6-7 and extracted with dichloromethane. Organic layer is decanted, dried over magnesium sulfate and solvent evaporated under reduced pressure to yield 10.3 g of the title compound that is used with no further purification. MS (m/z): 155 (M+1).

### Preparation 14: Methyl 2, 3-difluorobenzoate

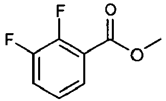

The compound of Preparation 14 is essentially prepared as described in Preparation 13 by using 2,3-difluorobenzoic acid and methyl alcohol in a 96% yield: ¹H-NMR (CDCl₃): 7.70 (m, 1H), 7.35 (m, 1H), 7.14 (m, 1H), 3.95 (s, 3H)

### Preparation 15: 2,3-Difluoro-N-methyl-benzamide

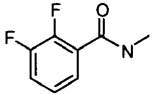

A mixture of 2,3-difluorobenzoic acid (1 g, 6.32 mmol) and thionyl chloride (9 mL, 123.53 mmol) is stirred and heated at reflux for 2h. The solvent is evaporated *in vacuo* and the residue is coevaporated with toluene. Once dried, the residue is dissolved in 5 mL of tetrahydrofuran, chilled at 0°C and monomethylamine (6.32 mL, 12.65 mmol) is added. After 20 min the reaction is quenched with water and extracted with ethyl acetate. The organic layer was separated, dried over magnesium sulfate and the solvent evaporated *in vacuo* to yield 790 mg of the title compound. MS (m/z): 172 (M+1)

### Preparation 16: 3-Fluoro-2-(4-formyl-3-methyl-pyrazol-1-yl)benzamide

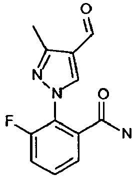

A mixture of 3-methyl-1H-pyrazole-4-carbaldehyde (15.25 g, 138.49 mmoles) and 2,3-difluorobenzamide (26.11 g, 166.19 mmoles) in dimethylformamide (228.75 mL) is cooled with ice/water bath and then potassium *tert*-butoxide (17.09 g, 152.34 mmoles) is added. The resulting mixture is stirred at 50°C for 20 hr. Reaction mixture is cooled down to room temperature. Then, ice/water (300 mL) is added and the mixture is extracted with ethyl acetate (3 x 200 mL). Organics are combined, dried over sodium sulfate and concentrated to afford a light brown oil containing 20% of the other pyrazole regioisomer. The residue is purified by silica gel chromatography using as eluent ethyl acetate/hexane to afford 17.4 g of the title compound. MS (m/z): 248 (M+1).

The compounds of Preparation 17-18 are prepared essentially as described in Preparation 16 from the corresponding 2,3-difluoro phenyl derivative.

| Prep. No. | Chemical name | Structure | Yield (%) | Physical data: MS (m/z) |
|---|---|---|---|---|
| 17 | 3-fluoro-2-(4-formyl-pyrazol-1-yl)benzamide | 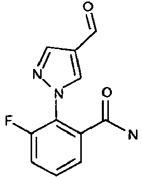 | 85 | 234 (M+1) |
| 18 | Methyl 3-fluoro-2-(4-formyl-3-methyl-pyrazol-1-yl)benzoate | 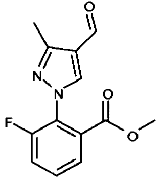 | 56 | 263 (M+1) |

### Preparation 19: 1-(2-Fluoro-6-nitro-phenyl)-3-methyl-1H-pyrazole-4-carbaldehyde

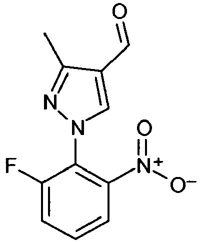

To a solution of 3-methyl-1H-pyrazole-4-carbaldehyde (1 g, 9.08 mmol) in acetonitrile (10 mL) is added potassium carbonate (1.76 g, 12.71 mmol) and 2,3-difluornitrobenzene (1.73 g, 10.90 mmol) and the mixture is stirred at room temperature overnight. Water is added and the organic phase is extracted with ethyl acetate. Organic layer is dried over sodium sulfate and the solvent evaporated under reduced pressure. The residue is purified by normal phase Isco chromatography using as eluent ethyl acetate/hexane (20-80%) to give a 62% yield of a mixture of regioisomers containing the title compound as major product that is used with no further purification.
¹H-NMR is consistent with desired structure, although mixture of regiosomers is detected: ¹H-NMR (MeOD): 9.98 (s, 1H), 8.65 (d, 1H, J= 1.6 Hz), 7.99-7.26 (m, 3H), 2.49 (s, 3H).

### Preparation 20: 3-Fluoro-2-(4-formyl-3-methyl-pyrazol-1-yl)benzonitrile

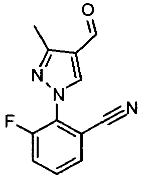

A mixture of 3-methyl-1H-pyrazole-4-carbaldehyde (5 g, 45.41 mmol), potassium carbonate (9.41 g, 68.11 mmol), 2,3-difluorobenzonitrile (6.06 mL, 54.5 mmol) and dimethylformamide (50 mL) is stirred at 100°C for 5 hr. and then at room temperature overnight. Water is added and a precipitated is formed. The precipitate is filtered. The aqueous solution filtered is extracted in ethyl acetate. Organic layer is washed with brine, dried over magnesium sulfate and solvent is evaporated. Both the solid precipitated and the solid recovered from organic layer after evaporation are combined and 10.4 g of the title compound is obtained and used with no further purification (the title compound is contaminated with the other pyrazole regioisomer in a ratio 90:10). MS (m/z): 230 (M+1).

The compounds of Preparation 21-27 are prepared essentially as described in Preparation 20 from the corresponding fluoro phenyl derivative.

| Prep. No. | Chemical name | Structure | Yield (%) | Physical data: MS (m/z) |
|---|---|---|---|---|
| 21 | 3-Chloro-2-(4-formyl-3-methyl-pyrazol-1-yl)benzamide | 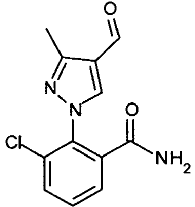 | 64 | 264 (M+1) |
| 22 | 3-Fluoro-2-(4-formyl-3-methyl-pyrazol-1-yl)-*N-*methyl-benzamide | 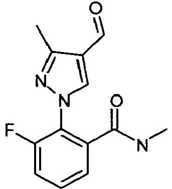 | 48 | 262 (M+1) |
| 23 | Methyl 3-bromo-2-(4-formyl-3-methyl-1H-pyrazol-1-yl)benzoate | 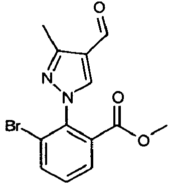 | 86 | 323, 325 (M+1, M+3) |
| 24 | 3-Fluoro-2-(4-formylpyrazol-1-yl)benzonitrile | 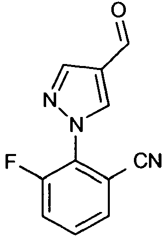 | 98 | 216 (M+1) |
| 25 | Ethyl 1-(2-acetyl-6-fluoro-phenyl)-3-methyl-pyrazole-4-carboxylate | 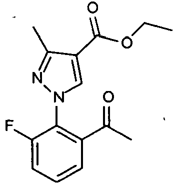 | 71 | 291 (M+1) |
| 26 | Ethyl 1-(2-chloro-6-formyl-phenyl)-3-methyl-pyrazole-4-carboxylate | 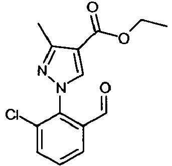 | 80 | 291 (M+1) |
| 27 | Ethyl 1-(2-fluoro-6-formyl-phenyl)-3-methyl-pyrazole-4-carboxylate | 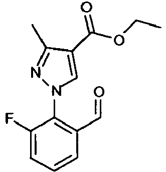 | 44 | 277 (M+1) |

### Preparation 28: Ethyl 1-[2-(dimethylaminomethyl)-6-fluoro-phenyl]-3-methyl-pyrazole-4-carboxylate

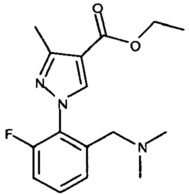

Compound is essentially prepared as described in Preparation 7 by using ethyl 1-(2-fluoro-6-formyl-phenyl)-3-methyl-pyrazole-4-carboxylate and dimethylamine. The residue is purified by normal phase Isco chromatography (eluent: dichloromethane/methanol) to give ethyl 1-[2-(dimethylaminomethyl)-6-fluoro-phenyl]-3-methyl-pyrazole-4-carboxylate in a 90% yield. MS (m/z): 306 (M+1)

### Preparation 29: [1-[2-(Dimethylaminomethyl)-6-fluoro-phenyl]-3-methyl-pyrazol-4-yl]methanol

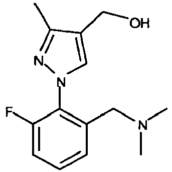

To a solution of ethyl 1-(2-dimethylaminomethyl-6-fluoro-phenyl)-3-methyl-1H-pyrazole-4-carboxylic acid ethyl ester (1.95 g, 6.39 mmol) in tetrahydrofuran (42.6 mL) cooled to 0°C and under nitrogen, 1 M lithium aluminum hydride in tetrahydrofuran (9.6 mL, 9.6 mmol) is added and the mixture is stirred at that temperature for 1 hr. Reaction mixture is treated at 0°C with 0.36 mL of water, 0.36 mL of 15% sodium hydroxide solution and finally 1.08 mL of water and stirred at room temperature for 15 min. The solid is filtered off and the solvent evaporated *in vacuo* to yield 1.68 g of the title compound that is used with no further purification. MS (m/z): 264 (M+1).

### Preparation 30: 1-[2-(Dimethylaminomethyl)-6-fluoro-phenyl]-3-methyl-pyrazole-4-carbaldehyde

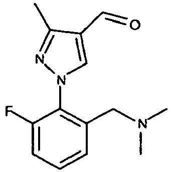

A mixture of [1-(2-dimethylaminomethyl-6-fluoro-phenyl)-3-methyl-1H-pyrazol-4-yl]-methanol (4.89 mmol; 1.43 g) and manganese (IV) oxide (4.25 g, 48.88 mmol) is stirred in dichloromethane (50 mL) at room temperature overnight. The reaction mixture is filtered over celite and the solvent evaporated under reduced pressure to yield 1.4 g of the title compound that is used without further purification. MS (m/z): 262 (M+1).

### Preparation 31: 1-[2-Fluoro-6-(morpholinomethyl)phenyl]-3-methyl-pyrazole-4-carbaldehyde

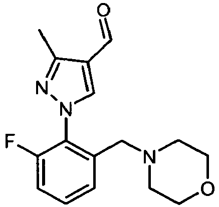

### 1. Ethyl 1-[2-fluoro-6-(morpholinomethyl)phenyl]-3-methyl-pyrazole-4-carboxylate

This compound is prepared essentially as described in Preparation 7 by using ethyl 1-(2-fluoro-6-formyl-phenyl)-3-methyl-pyrazole-4-carboxylate and morpholine. MS (m/z): 348 (M+1)

### 2. [1-[2-Fluoro-6-(morpholinomethyl)phenyl]-3-methyl-pyrazol-4-yl]methanol

This compound is essentially prepared as described in Preparation 29 by using ethyl 1-[2-fluoro-6-(morpholinomethyl)phenyl]-3-methyl-pyrazole-4-carboxylate. MS (m/z): 306 (M+1)

### 3. 1-[2-Fluoro-6-(morpholinomethyl)phenyl]-3-methyl-pyrazole-4-carbaldehyde

3,3,3-Triacetoxy-3-iodophthalide (0.42 g, 0.96 mmol) is added to a solution of [1-(2-fluoro-6-morpholin-4-ylmethyl-phenyl)-3-methyl-1H-pyrazol-4-yl]-methanol (0.24 g, 0.8 mmol) in dichloromethane (3 mL) at room temperature. After one hour the reaction is quenched by addition of 2N sodium carbonate solution and the compound is extracted in dichloromethane. The organic layer is separated, dried over magnesium sulfate and the solvent evaporated *in vacuo* to afford the title compound in 99% yield that is used with no further purification. MS (m/z): 304 (M+1).

### Preparation 32: 1-[2-Chloro-6-(dimethylaminomethyl)phenyl]-3-methyl-pyrazole-4-carbaldehyde

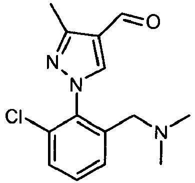

The title compound is prepared using a method essentially as described in preparation 31 using dimethylamine and ethyl 1-(2-chloro-6-formyl-phenyl)-3-methylpyrazole-4-carboxylate (68% yield). MS (m/z): 278 (M+1).

### Preparation 33: tert-Butyl N-cyclopropyl-N-[[3-fluoro-2-(4-formyl-3-methyl-pyrazol-1-yl)phenyl]methyl]carbamate

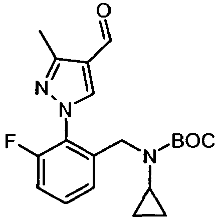

### 1. Ethyl 1-[2-[(cyclopropylamino)methyl]-6-fluoro-phenyl]-3-methyl-pyrazole-4-carboxylate

The following compound is prepared essentially as described in Preparation 7 by using ethyl 1-(2-fluoro-6-formyl-phenyl)-3-methyl-pyrazole-4-carboxylate and cyclopropylamine. MS (m/z): 318 (M+1)

### 2. Ethyl 1-[2-[(tert-butoxycarbonyl(cyclopropyl)amino)methyl]-6-fluoro-phenyl]-3-methyl-pyrazole-4-carboxylate

To a solution of 1-(2-cyclopropylaminomethyl-6-fluoro-phenyl)-3-methyl-1H-pyrazole-4-carboxylic acid ethyl ester (330.00 mg, 1.04 mmol) in dichloromethane (3 mL) is added at room temperature tert-butoxycarbonyl tert-butyl carbonate (226.94 mg, 1.04 mmol) and triethylamine (115.52 mg, 1.14 mmol). After 1 hr. water is added and the compound is extracted with DCM. The organic layer is separated, dried over magnesium sulfate and the solvent evaporated under reduced pressure. The crude is purified with a short silica gel plug and hexane/ethyl acetate 5:1 as eluent to afford 349 mg of ethyl 1-[2-[(tert butoxycarbonyl(cyclopropyl)amino)methyl]-6-fluoro-phenyl]-3-methyl-pyrazole-4-carboxylate. MS (m/z): 418 (M+1)

### 3. tert-Butyl N-cyclopropyl-N-[[3-fluoro-2-[4-(hydroxymethyl)-3-methyl-pyrazol-1-yl]phenyl]methyl]carbamate

The following compound is prepared essentially as described in Preparation 29 at 0°C by using ethyl 1-[2-[(tert-butoxycarbonyl(cyclopropyl)amino)methyl]-6-fluoro-phenyl]-3-methyl-pyrazole-4-carboxylate in a 92% yield. MS (m/z): 376 (M+1)

### 4. tert-Butyl N-cyclopropyl-N-[[3-fluoro-2-(4-formyl-3-methyl-pyrazol-1-yl)phenyl]methyl]carbamate

The following compound is essentially prepared as described in Preparation 31, step 3 (final oxidation step) by using *tert*-butyl *N*-cyclopropyl-*N*-[[3-fluoro-2-[4-(hydroxymethyl)-3-methyl-pyrazol-1-yl]phenyl]methyl]carbamate. MS (m/z): 374 (M+1)

### Preparation 34: 1-[2-Fluoro-6-(1-methylimidazol-2-yl)phenyl]-3-methyl-pyrazole-4-carbaldehyde

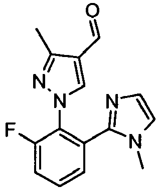

### 1. 1-(2-Cyano-6-fluoro-phenyl)-3-methyl-1H-pyrazole-4-carboxylic acid ethyl ester

A mixture of 3-methyl-1H-pyrazole-4-carboxylic acid ethyl ester (1.25 g, 8.11 mmol), potassium carbonate (1.68 g, 12.16 mmol), 2,3-difluorobenzonitrile (1.08 mL, 9.73 mmol) in dimethylformamide (12 mL) is heated at 100°C with the aid of a magnetic stirred. After 2.5 hr. the reaction mixture is treated with water and extracted with ethyl acetate. The organic layer is decanted, washed with brine, dried over magnesium sulfate and the solvent evaporated under reduced pressure to give 2.3 g of 1-(2-cyano-6-fluorophenyl)-3-methyl-1H-pyrazole-4-carboxylic acid ethyl ester (this compound is contaminated with the other pyrazole regioisomer in a ratio 75:25). MS (m/z): 274 (M+1).

### 2. Ethyl 1-[2-fluoro-6-(1-methyl-4,5-dihydroimidazol-2-yl)phenyl]-3-methyl-pyrazole-4-carboxylate

A capped vial is charged with ethyl 1-(2-cyano-6-fluoro-phenyl)-3-methylpyrazole-4-carboxylate (1.97 g, 7.21 mmol) (contaminated with the other pyrazole regioisomer in a ratio of 75:25, 1,2-ethanediamine, *N*-methyl- (6 mL, 68.02 mmol) and phosphorus pentasulfide (229 mg, 1,01 mmol) and the mixture is stirred at 110°C for 30 min and then allow to reach rt. Solvent is evaporated *in vacuo* and the residue purified by normal phase Isco chromatography using dichloromethane/2M ammonia in methanol from 95/5 to 85/15 as eluent to yield 2.11 g of ethyl 1-[2-fluoro-6-(1-methyl-4,5-dihydroimidazol-2-yl)phenyl]-3-methyl-pyrazole-4-carboxylate (contaminated with the other pyrazole regioisomer in a ratio 75:25). MS (m/z): 331 (M+1).

### 3. Ethyl 1-[2-fluoro-6-(1-methylimidazol-2-yl)phenyl]-3-methyl-pyrazole-4-carboxylate

Potassium permanganate (1.58 g, 10 mmmol) and montmorillonite K-10 (3.16 g) are grounded together in a mortar until a fine homogeneous powder is obtained. KMnO4-montmorillonite K-10 (3.2 g, 6.78 mmol) is added portionwise to a solution of ethyl 1-[2-fluoro-6-(1-methyl-4,5-dihydroimidazol-2-yl)phenyl]-3-methyl-pyrazole-4-carboxylate (1.12 g, 3.39 mmol) (contaminated with the other pyrazole regioisomer in a ratio 75:25) in acetonitrile (84.76 mL, 1.62 moles). The mixture is stirred at room temperature for 6.5 hr. and more KMnO₄-montmorillonite K-10 (0.8 g, 1.69 mmol) is added portionwise and the mixture stirred at room temperature overnight. Ethanol is added and stirred for additional 20 min. Then the reaction mixture is filtered through a short pad of celite and the solid material is washed with acetonitrile. The solvent is evaporated under reduced pressure and the crude mixture is purified normal phase Isco chromatography using ethyl acetate as eluent to yield 518 mg of 1-[2-fluoro-6-(1-methylimidazol-2-yl)phenyl]-3-methyl-pyrazole-4-carboxylate (contaminated with the other pyrazole regioisomer in a ratio 75:25). MS (m/z): 329 (M+1).

### 4. [1-[2-Fluoro-6-(1-methylimidazol-2-yl)phenyl]-3-methyl-pyrazol-4-yl]methanol

This compound is essentially prepared as described in Preparation 29 by using ethyl 1-[2-fluoro-6-(1-methylimidazol-2-yl)phenyl]-3-methyl-pyrazole-4-carboxylate (contaminated with the other pyrazole regioisomer in a ratio 75:25) in 99% yield. MS (m/z): 287 (M+1).

### 5. 1-[2-Fluoro-6-(1-methylimidazol-2-yl)phenyl]-3-methyl-pyrazole-4-carbaldehyde

The following compound is essentially prepared as described in Preparation 30 by using [1-[2-fluoro-6-(1-methylimidazol-2-yl)phenyl]-3-methyl-pyrazol-4-yl]methanol (contaminated with the other pyrazole regioisomer in a ratio 75:25). Residue is purified by normal phase Isco chromatography using ethyl acetate as eluent to give 64% yield of the title compound (contaminated with the other pyrazole regioisomer in a ratio 75:25). MS (m/z): 285 (M+1).

### Preparation 35: Methyl N-[3-fluoro-2-(4-formyl-3-methyl-pyrazol-1-yl)phenyl]carbamate

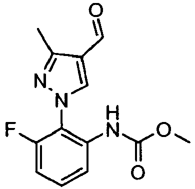

### 1. 1-(2-Amino-6-fluoro-phenyl)-3-methyl-pyrazole-4-carbaldehyde

A mixture of 1-(2-fluoro-6-nitro-phenyl)-3-methyl-1H-pyrazole-4-carbaldehyde (620 mg; 2.49 mmol) (as major compound in a mixture of regioisomers in the pyrazole) and Iron (1.40 g) in ethanol (5.1 mL) and water (5.1 mL) with few drops of acetic acid is heated at 90°C for 2h. After that time, it is filtered over celite, and eluted with more ethanol. Mixture is concentrated under vacuum, basified with sodium bicarbonate (saturated aqueous solution) and extracted with dichloromethane. Organic layer is decanted, dried over magnesium sulfate and solvent evaporated under reduced pressure to give 500 mg of the title compound, as major product in a mixture of regioisomers in the pyrazole, that is used without further purification. MS (m/z): 220 (M+1).

### 2. Methyl N-[3-fluoro-2-(4-formyl-3-methyl-pyrazol-1-yl)phenyl]carbamate

To a solution of 1-(2-amino-6-fluoro-phenyl)-3-methyl-1H-pyrazole-4-carbaldehyde (500 mg, 2.28 mmol) (as major compound in a mixture of regioisomers in the pyrazole) in dichloromethane (15.21 mL), pyridine (553.31 µL) is added. Then, methyl chloroformate (194.17 µL) is added dropwise at 0°C and the mixture is stirred at room temperature for 30 min. Water is added and the mixture is extracted with dichloromethane. Organic layer is decanted, dried over magnesium sulfate and solvent evaporated under reduced pressure. The residue is purified by normal phase Isco chromatography using as eluent ethyl acetate and hexane to give 418 mg of the title compound. MS (m/z): 278 (M+1).

### Preparation 36: Methyl N-[3-fluoro-2-(4-formyl-3-methyl-pyrazol-1-yl)phenyl]-N-methyl-carbamate

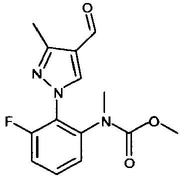

To a solution of methyl *N*-[3-fluoro-2-(4-formyl-3-methyl-pyrazol-1-yl)phenyl]carbamate (335 mg, 1.2 mmol) (as major compound in a mixture of regioisomers in the pyrazole) in tetrahydrofuran (6 mL) under nitrogen atmosphere and cooled to 0°C, sodium hydride (60% in mineral oil) (58.3 mg) is added. Then, methyl iodide (0.4 mL) is added and the reaction mixture is stirred at 0°C for 1 hour. After that time, water is added and the mixture is extracted with ethyl acetate. Organic layer is decanted, dried over sodium sulfate and solvent evaporated. The residue is purified by normal phase Isco chromatography using as eluent ethyl acetate and hexane to give 287 mg of the title compound, as major product in a mixture of regioisomers in the pyrazole, that is used without further purification. MS (m/z): 292 (M+1).

### Preparation 37: Ethyl 1-[2-fluoro-6-(hydroxymethyl)phenyl]-3-methyl-pyrazole-4-carboxylate

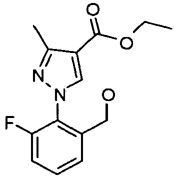

To a solution of 1-(2-fluoro-6-formyl-phenyl)-3-methyl-1H-pyrazole-4-carboxylic acid ethyl ester (1,5 g, 5,43 mmol) in methanol (10 mL) is added sodium borohydride (246,50 mg, 6.52 mmol) at rt. Solution is stirred for 30 min and then the solvent is evaporated under reduced pressure and the residue is solved in ethyl acetate, washed with saturated aqueous solution of sodium bicarbonate, water and brine. The organic phase is dried over magnesium sulfate, filtered and concentrated to afford the title compound in a 99% yield that is used with no further purification. MS (m/z): 279 (M+1).

### Preparation 38: 1-[2-Fluoro-6-(methoxymethyl)phenyl]-3-methyl-pyrazole-4-carbaldehyde

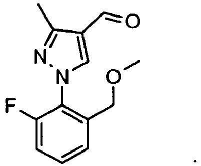

### 1. Ethyl 1-[2-fluoro-6-(methoxymethyl)phenyl]-3-methyl-pyrazole-4-carboxylate

To a solution of ethyl 1-[2-fluoro-6-(hydroxymethyl)phenyl]-3-methyl-pyrazole-4-carboxylate (1.2 g, 4.34 mmol) in 10 mL of tetrahydrofuran is added 60% sodium hydride (0.21 g, 5.21 mmol) at 0°C under nitrogen atmosphere. Solution is stirred at 0°C for 1 hr. Methyl iodide (0.81 mL, 13.02 mmol) is added over the solution. The mixture is stirred at room temperature overnight. The mixture is quenched by addition of water and the crude is extracted with ethyl acetate. The organic layer is separated, dried over magnesium sulfate and solvent evaporated *in vacuo.* The residue is purified by normal phase Isco chromatography eluting with 10% dichloromethane/methanol to afford ethyl 1-[2-fluoro-6-(methoxymethyl)phenyl]-3-methyl-pyrazole-4-carboxylate. As compound is not completely pure it is further purified by HPLC to afford the compound in 18% yield. MS (m/z): 293.1 (M+1)

### 2. [1-[2-Fluoro-6-(methoxymethyl)phenyl]-3-methyl-pyrazol-4-yl]methanol

This compound is prepared essentially as described in Preparation 29 by using ethyl 1-[2-fluoro-6-(methoxymethyl)phenyl]-3-methyl-pyrazole-4-carboxylate in a 92% yield. MS (m/z): 251 (M+1)

### 3. 1-[2-Fluoro-6-(methoxymethyl)phenyl]-3-methyl-pyrazole-4-carbaldehyde

The following compound is essentially prepared as described in Preparation 30 by using [1-[2-fluoro-6-(methoxymethyl)phenyl]-3-methyl-pyrazol-4-yl]methanol in a 99% yield. MS (m/z): 249 (M+1).

### Preparation 39: [3-Fluoro-2-(4-formyl-3-methyl-pyrazol-1-yl)phenyl]methyl N-methylcarbamate

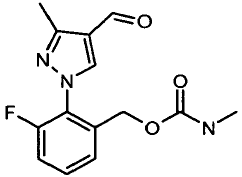

### 1. Ethyl 1-[2-fluoro-6-(methylcarbamoyloxymethyl)phenyl]-3-methyl-pyrazole-4-carboxylate

To a solution of 1-(2-fluoro-6-hydroxymethyl-phenyl)-3-methyl-1H-pyrazole-4-carboxylic acid ethyl ester (300 mg, 1.08 mmol) in dichloromethane (3 mL) is added methylisocyanate (71.55 µL, 1.19 mmol) at room temperature. After 1 hr. , more methylisocyanate (1 eq) and triethylamine (1 eq) are added. One hour later, the reaction is quenched with saturated aqueous solution of sodium bicarbonate and extracted with dichloromethane. The organic layer is separated, dried over magnesium sulfate and solvent evaporated *in vacuo* to afford 360 mg of ethyl 1-[2-fluoro-6-(methylcarbamoyloxymethyl)phenyl]-3-methyl-pyrazole-4-carboxylate that is used with no further purification. MS (m/z): 336 (M+1).

### 2. [3-Fluoro-2-[4-(hydroxymethyl)-3-methyl-pyrazol-1-yl]phenyl]methyl N-methylcarbamate

This compound is prepared essentially as described in Preparation 29 by using ethyl 1-[2-fluoro-6-(methylcarbamoyloxymethyl)phenyl]-3-methyl-pyrazole-4-carboxylate in a 82% yield. MS (m/z): 294 (M+1)

### 3. [3-Fluoro-2-(4-formyl-3-methyl-pyrazol-1-yl)phenyl]methyl N-methylcarbamate

This compound is prepared essentially as described in Preparation 31, step 3 (final oxidation step) by using [3-fluoro-2-[4-(hydroxymethyl)-3-methyl-pyrazol-1-yl]phenyl]methyl *N*-methylcarbamate. The residue is purified by normal phase Isco chromatography using hexane and ethyl acetate (0 to 70% in ethyl acetate) as eluent to give 169 mg of the title compound. MS (m/z): 292 (M+1)

### Preparation 40: 1-[2-(Dimethylaminomethyl)phenyl]-3-methyl-pyrazole-4-carbaldehyde

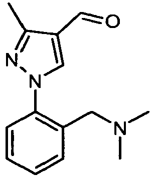

### 1. [1-[2-(Dimethylaminomethyl)phenyl]-3-methyl-pyrazol-4-yl]methanol

[1-(2-Chloro-6-dimethylaminomethyl-phenyl)-3-methyl-H-pyrazol-4-yl]-methanol (777.00 mg, 2.78 mmol) is hydrogenated in ethyl acetate (15 mL) with Pd(C)10% (77 mg) with an hydrogen balloon. After 1.5 hr. the mixture is filtered through a plug of celite using methanol as eluent. The solvent is evaporated and the residue was basified with saturated aqueous solution of sodium bicarbonate and extracted with dichloromethane. The organic layer is separated, dried over magnesium sulfate and solvent evaporated *in vacuo.* The residue is purified by normal phase Isco chromatography using 2N ammonia in methanol and dichloromethane (5 to 7% in methanol) as eluent to afford 531 mg of [1-[2-(dimethylaminomethyl)phenyl]-3-methyl-pyrazol-4-yl]methanol. MS (m/z): 246 (M+1).

### 2. 1-[2-(Dimethylaminomethyl)phenyl]-3-methyl-pyrazole-4-carbaldehyde

This compound is prepared essentially as described in Preparation 31,step 3 (final oxidation step) by using [1-[2-(dimethylaminomethyl)phenyl]-3-methyl-pyrazol-4-yl]methanol. MS (m/z): 244 (M+1)

### Preparation 41: Methyl 3-cyclopropyl-2-(4-formyl-3-methyl-1H-pyrazol-1-yl)benzoate

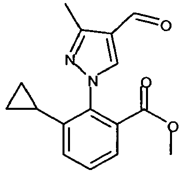

To a screw-cap test tube containing a mixture of methyl 3-bromo-2-(4-formyl-3-methyl-1H-pyrazol-1-yl)benzoate (412 mg, 1.3 mmol), cyclopropylboronic acid (142 mg, 1.7 mmol), potassium phosphate (947 mg, 4.5 mmol) and tricyclohexylphosphine (36 mg, 0.13 mmol) in a mixture of toluene (5.7 mL) and water (0.28 mL) is added under nitrogen palladium acetate (14 mg, 0.06 mmol). The reaction tube is quickly sealed (caution: build-up of pressure possible; use a safety shield) and stirred in a preheated oil bath at 100°C for 18 hr. with the aid of a magnetic stirrer. The mixture is diluted with water and extracted with ethyl acetate. The organic layer is separated, dried over magnesium sulfate, filtered and the solvent evaporated *in vacuo.* The resulting residue is purified by normal phase Isco chromatography eluting with hexane:ethanol (gradient from 2 to 15% in ethanol) to give 96 mg of the title compound. MS (m/z): 285 (M+1).

### Preparation 42: 1-(2,6-Dimethylphenyl)-3-methyl-1H-pyrazole-4-carbaldehyde

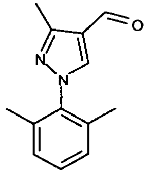

A screw-cap test tube containing molecular sieves (4A) and a solution of 3-methyl-1H-pyrazole-4-carbaldehyde (100 mg, 0.91 mmol), 2,6-dimethylphenylboronic acid (150 mg, 1 mmol), copper(II) acetate (247 mg, 1.36 mmol) and pyridine (147 µl, 1.8 mmol) in dry dichloromethane (4.5 mL) is shaked at room temperature for 48 hr. The mixture is filtered over celite, washed with methanol and the solvent evaporated *in vacuo.* Resulting product is purified by silica gel using normal phase Isco chromatography eluting with hexane: acetone (gradient from 5 to 30% in acetone) to give 67 mg of the title compound. MS (m/z): 215 (M+1)

### Preparation 43: Ethyl 1-(2,6-difluorophenyl)-4-formyl-pyrazole-3-carboxylate

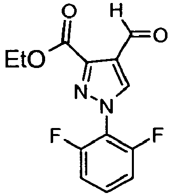

### 1. Ethyl 2-[(2,6-difluorophenyl)hydrazono]propanoate

To a solution of (2,6-difluorophenyl)hydrazine hydrochloride (3.0 g, 16.6 mmol) in water (50 mL) is added methylpyruvate (2.32 g, 19.9 mmol) and sodium acetate (6.8 g, 50.0 mmol) at room temperature. The mixture is stirred for 2 hours. After completion, the precipitated solid is filtered and washed with water to yield 3.0 g (74%) of ethyl 2-[(2,6-difluorophenyl)hydrazono]propanoate. MS (m/z): 243 (M+1).

### 2. Ethyl 1-(2,6-difluorophenyl)-4-formyl-pyrazole-3-carboxylate

To a solution of ethyl 2-[(2,6-difluorophenyl)hydrazono]propanoate (3.0 g, 12.3 mmol) in dimethylformamide (20 mL) is added slowly phosphorus(V) oxychloride (9.6 mL, 99.1 mmol) at 0 °C. The mixture is heated to 60 °C for 5 hr. After completion, the reaction mixture is cooled to 0 °C, neutralized with aqueous saturated sodium bicarbonate solution (75 mL) and extracted with ethyl acetate (3 x 30 mL). The combined organic extracts are dried over sodium sulfate and concentrated *in vacuo.* The crude mixture is purified by column chromatography over silica gel eluting with hexane/ethyl acetate (30:70) to yield 2.5 g (73%) of the title compound. MS (m/z): 281 (M+1).

### Preparation 44: Ethyl 1-(2-fluorophenyl)-4-formyl-pyrazole-3-carboxylate

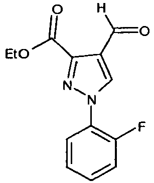

The title compound is prepared using a method essentially as described in Preparation 43 by using 2-fluorophenylhydrazine hydrochloride (56% yield). MS (m/z): 263 (M+1).

### Preparation 45: 1-(2-Fluorophenyl)-3-(methoxymethyl)pyrazole-4-carbaldehyde

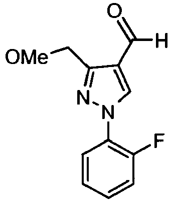

### 1. tert-Butyl 3-[(2-fluorophenyl)hydrazono]butanoate

To a solution of ethanol (15 mL) and pyridine (65 mL) are added tert-butyl 3-oxobutanoate (5.0 g, 31.6 mmol) and (2-fluorophenyl)hydrazine (4.4 g, 34.9 mmol) at room temperature and stirred for 16 hr. After completion, the reaction mixture is partitioned between ether (100 mL) and 1N hydrochloric acid (50 mL). The organic layer is washed with water, brine, dried over sodium sulfate and concentrated *in vacuo.* The crude mixture is purified by column chromatography over silica gel eluting with hexane/ethyl acetate (90:10) to yield 8 g (95%) of *tert*-butyl 3-[(2-fluorophenyl)hydrazono]butanoate. MS (m/z): 267 (M+1).

### 2. tert-Butyl 1-(2-fluorophenyl)-3-methyl-pyrazole-4-carboxylate

To a solution of *tert*-butyl 3-[(2-fluorophenyl)hydrazono]butanoate (8.0 g, 30 mmol) in toluene (100 mL) is added *N,N*-dimethylformamide dimethylacetal (3.9 mL, 30 mmol) and then heated to 110 °C for 14 hr. After completion, the reaction mixture is concentrated *in vacuo* and purified by column chromatography over silica gel eluting with hexane/ethyl acetate (95:5) to yield *tert*-butyl 1-(2-fluorophenyl)-3-methyl-pyrazole-4-carboxylate. MS (m/z): 277 (M+1).

### 3. tert-Butyl 3-(bromomethyl)-1-(2-fluorophenyl)pyrazole-4-carboxylate

A solution of *tert*-butyl 1-(2-fluorophenyl)-3-methyl-pyrazole-4-carboxylate (1.0 g, 3.6 mmol) and benzoylperoxide (0.08 g, 0.36 mmol) in carbon tetrachloride (30 mL) is irradiated using 100 W lamp and heated under reflux. Then *N*-bromosuccinimide is added (0.58 g, 3.2 mmol) in four equal intervals over 7 hr. The reaction mixture is further refluxed for 2 hr. After completion, the reaction mixture is cooled to room temperature and partitioned between water (25 mL) and dichloromethane (25 mL). The aqueous phase is extracted with dichloromethane (3 x 20 mL) and the combined organic extracts are dried over sodium sulfate and concentrated *in vacuo* to give *tert*-butyl 3-(bromomethyl)-1-(2-fluorophenyl)pyrazole-4-carboxylate which is used as such for the next step. MS (m/z): 355, 357 (M+1, M+3).

### 4. 1-(2-Fluorophenyl)-3-(methoxymethyl)pyrazole-4-carboxylic acid

To a solution of *tert*-butyl 3-(bromomethyl)-1-(2-fluorophenyl)pyrazole-4-carboxylate (1.5 g, 4.2 mmol) in methanol (30 mL) are added sodium methoxide (6.86 g, 27.5 mL, 25 wt % in methanol, 127 mmol) and the mixture is stirred at room temperature for 5 hr. After completion, the reaction mixture is concentrated *in vacuo* to give the crude mixture that is used as such. To a solution of this crude mixture (1.5 g, 4.9 mmol) in methanol (10 mL) is added aqueous 10 % sodium hydroxide (10 mL) and stirred at room temperature for 24 hours. After completion, the reaction mixture is concentrated *in vacuo.* Water is added to the residue and pH is adjusted to 5 with 1N hydrochloric acid and extracted with ethyl acetate (2 x 50 mL). The combined organic extracts are dried over sodium sulfate and concentrated *in vacuo* to yield 0.8 g (66%) of 1-(2-fluorophenyl)-3-(methoxymethyl)pyrazole-4-carboxylic acid. MS (m/z): 251 (M+1).

### 5. [1-(2-Fluorophenyl)-3-(methoxymethyl)pyrazol-4-yl]methanol

To a solution of 1-(2-fluorophenyl)-3-(methoxymethyl)pyrazole-4-carboxylic acid (0.8 g, 3.2 mmol) in tetrahydrofuran (8 mL) and toluene (8 mL) is added 5.0 M borane dimethylsulfide complex in ether (3.80 mL, 19.2 mmol) at 0 °C and stirred at room temperature for 16 hr. After completion, the reaction mixture is quenched with aqueous ammonium chloride (25 mL) and extracted with ethyl acetate (3 x 25 mL). The combined organic extracts are dried over sodium sulfate and concentrated *in vacuo.* The crude mixture is purified by column chromatography over silica gel eluting with dichloromethane/methanol (99:1) to yield 0.3 g (40%) of [1-(2-fluorophenyl)-3-(methoxymethyl)pyrazol-4-yl]methanol. MS (m/z): 237 (M+1).

### 6. 1-(2-Fluorophenyl)-3-(methoxymethyl)pyrazole-4-carbaldehyde

To a solution of [1-(2-fluorophenyl)-3-(methoxymethyl)pyrazol-4-yl]methanol (0.30 g, 1.2 mmol) in dichloromethane (10 mL) is added Dess-Martin periodinane (0.65 g, 1.5 mmol) at 0 °C and continued the stirring for 4 hr. at 0 °C. After completion, the reaction mixture is quenched with aqueous sodium bicarbonate (15 mL) and extracted with dichloromethane (2 x 25 mL). The combined organic extracts are dried over sodium sulfate and concentrated *in vacuo.* The crude mixture is purified by column chromatography over silica gel eluting with dichloromethane/methanol (99.5:0.5) to yield 0.28 g (96%) of the title compound. MS (m/z): 235 (M+1).

### Example 1: [2-[4-[(2-Chloro-4,4-difluoro-spiro[5H-thieno[2,3-c]pyran-7,4'-piperidine ]-1'-yl)methyl]-3-methyl-pyrazol-1-yl]-3-fluoro-phenyl]methanol (L)-Tartrate

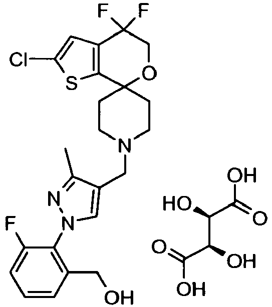

### 1. Methyl 2-[4-[(2-chloro-4,4-difluoro-spiro[5H-thieno[2.3-c]pyran-7,4'-piperidine]-1'-yl)methyl]-3-methyl-pyrazol-1-yl]-3-fluoro-benzoate

A solution of methyl 3-fluoro-2-(4-formyl-3-methyl-pyrazol-1-yl)benzoate (17 g, 64.83 mmol) and 2-chloro-4,4-difluoro-spiro[5H-thieno[2,3-c]pyran-7,4'-piperidine] (19.95 g, 71.31 mmol) in tetrahydrofuran (170 mL) is stirred at room temperature for 1 hr. Then sodium triacetoxyborohydride (16.49 g, 77.79 mmol) is added and the mixture is stirred at room temperature overnight. The reaction is quenched with saturated aqueous solution of sodium bicarbonate (200 mL) and the resulting phases are separated. The aqueous layer is extracted with ethyl acetate (2 x 50 mL). The organics are washed with brine (100 mL), dried over sodium sulfate and evaporated *in vacuo.* Crude is dissolved in methyl *tert-butyl* ether and a beige solid precipitated. Solid is filtered and discarded. Solution is concentrated and purified by silica gel chromatography using hexane/ethyl acetate mixtures to give 25 g of methyl 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-benzoate in a 73% yield. MS (m/z): 526 (M+1)

### 2. [2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-phenyl]methanol (L)-Tartrate

To a solution of methyl 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-benzoate (30 g, 57.04 mmol) in tetrahydrofuran (240 mL) under nitrogen at -20°C is added 1M lithium aluminum hydride in tetrahydrofuran (45.63 mL, 45.63 mmol). The cold bath is removed allowing the reaction mixture to reach 0°C (30 min). Water (2 mL) is added droppwise cautiously (gas evolution!), followed by 2N sodium hydroxide (2 mL) and water (6 mL). The resulting suspension is stirred at room temperature for 30 min. Suspension is filtered and solid is washed with ethyl acetate (20 mL). Filtrated is dried over sodium sulfate and concentrated to give 28 g of [2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]methanol. MS (m/z): 498 (M+1).

A solution of [2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]methanol (384 mg, 0.77 mmol) in 4 mL of methanol is added to a solution of (*L*)-tartaric acid (115.7 mg, 0.77 mmol) in 4 mL of methanol. After stirring for a few minutes the solvent is evaporated and the residue is dried *in vacuo* overnight to give [2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]methanol (*L*)-Tartrate in a quantitative yield. MS (m/z): 498 (M+1).

### Example 2: 2-Chloro-1'-[[1-(2,6-difluorophenyl)-3-methyl-pyrazol-4-yl]methyl]-4,4-difluoro-spiro[5H-thieno[2,3-c]pyran-7,4'-piperidine] (L)-Tartrate

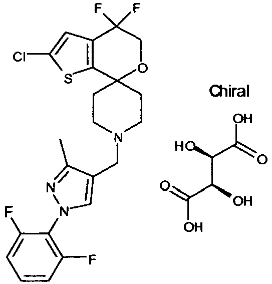

To a solution of 2-chloro-4,4-difluoro-spiro[5H-thieno[2,3-c]pyran-7,4'-piperidine] (7.14 g, 25.52 mmoles) in 1,2-dichloroethane (54 mL) is added 1-(2,6-difluoro-phenyl)-3-methyl-1*H*-pyrazole-4-carbaldehyde (5.40 g, 24.30 mmoles) and the mixture is stirred at room temperature for 30 min. Then, powdered sodium triacetoxiborohydride (9.27 g, 43.75 mmoles) is added (internal temperature 25-35°) and the resulting suspension is stirred at room temperature for 2 hr. A water/ice mixture (30 mL) is added in portions with stirring. The phases are separated. The aqueous phase is extracted with tert-butyl methyl ether (50 mL). Combined organic layers are washed with 50% saturated aqueous solution of sodium bicarbonate (50 mL), water (50 mL) and 50% brine (50 mL), dried over magnesium sulfate and concentrated to afford a thick oil which is purified via silica gel chromatography using dichloromethane/methanol (97:3) as eluent to give 7.1 g of 2-chloro-1'-[[1-(2,6-difluorophenyl)-3-methyl-pyrazol-4-yl]methyl]4,4-difluoro-spiro[5H-thieno[2,3-c]pyran-7,4'-piperidine]. MS (m/z): 486 (M+1).

The tartrate salt is essentially prepared as described in Example 1. MS (m/z): 486 (M+1).

The compounds of Example 3-21 are prepared essentially as described in Example 2 from de corresponding aldehydes.

| Ex. No. | Chemical name | Structure | Yield (%) | Physical data: MS (m/z) |
|---|---|---|---|---|
| 3 | methyl *N*-[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]carbamate (*L*)-Tartrate | 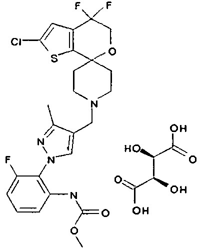 | 55^{a} | 541 (M+1) |
| 4 | 2-chloro-4,4-difluoro-1'-[[1-[2-fluoro-6-(morpholinomethyl)phenyl]-3-methyl-pyrazol-4-yl]methyl]spiro[5H-thieno[2,3-c]pyran-7,4'-piperidine] (*L*)-Tartrate | 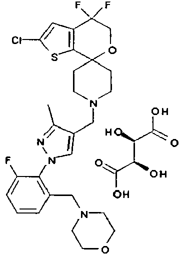 | 42 | 567 (M+1) |
| 5 | 1-[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]-*N,N-*dimethyl-methanamine (*L*)-Tartrate | 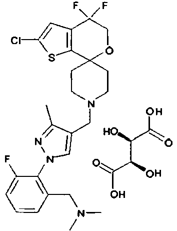 | 16 | 525 (M+1) |
| 6 | 1-[3-chloro-2-[4-[(2-chloro-4,4-difluoro-spiro[5H-thieno[2,3-c]pyran-7,4'-piperidine]-1'-yl)methyl]-3-methyl-pyrazol-1-yl]phenyl]-*N,N*-dimethyl-methanamine (*L*)-Tartrate | 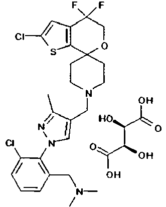 | 62 | 541 (M+1) |
| 7 | 1-[2-[4-[(2-chloro-4,4-difluoro-spiro[5H-thieno[2,3-c]pyran-7,4'-piperidine]-1'-yl)methyl]-3-methyl-pyrazol-1-yl]phenyl]-*N,N*-dimethyl-methanamine (*L*)-Tartrate | 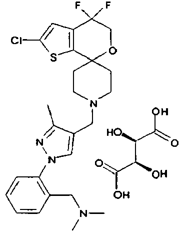 | 40 | 508 (M+1) |
| 8 | [2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]methyl *N-*methylcarbamate (*L*)-Tartrate | 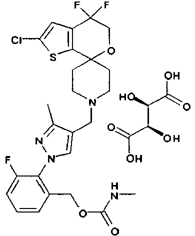 | 49 | 555 (M+1) |
| 9 | 3-fluoro-2-[4-[(2-fluorospiro[4,5-dihydrothieno[2,3-c]pyran-7,4'-piperidine]-1'-yl)methyl]-3-methyl-pyrazol-1-yl]benzamide (*L*)-Tartrate | 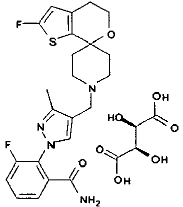 | 82 | 459 (M+1) |
| 10 | 1'-[[1-(2,6-difluorophenyl)-3-methyl-pyrazol-4-yl]methyl]-2-fluoro-spiro[4,5-dihydrothieno[2,3-c]pyran-7,4'-piperidine] (*L*)-Tartrate | 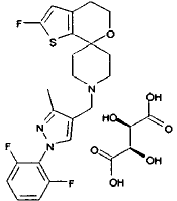 | 46 | 434 (M+1) |
| 11 | 3-fluoro-2-[4-[(2-fluorospiro[4,5-dihydrothieno[2,3-c]pyran-7,4'-piperidine]-1'-yl)methyl]-3-methyl-pyrazol-1-yl]benzonitrile (*L*)-Tartrate | 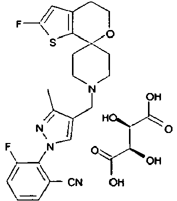 | 50 | 441 (M+1) |
| 12 | 1-[3-fluoro-2-[4-[(2-fluorospiro[4,5-dihydrothieno[2,3-c]pyran-7,4'-piperidine]-1'-yl)methyl]-3-methyl-pyrazol-1-yl]phenyl]-*N,N-*dimethyl-methanamine (*L*)-Tartrate | 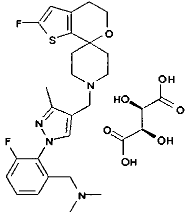 | 64 | 473 (M+1) |
| 13 | 3-fluoro-2-[4-[(2-fluorospiro[4,5-dihydrothieno[2,3-c]pyran-7,4'-piperidine]-1'-yl)methyl]-3-methyl-pyrazol-1-yl]-*N*-methyl-benzamide (*L*)-Tartrate | 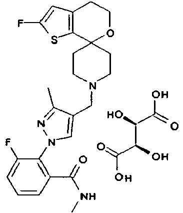 | 50 | 473 (M+1) |
| 14 | 3-fluoro-2-[4-[(2-fluorospiro[4,5-dihydrothieno[2,3-c]pyran-7,4'-piperidine]-1'-yl)methyl]pyrazol-1-yl]benzamide (*L*)-Tartrate | 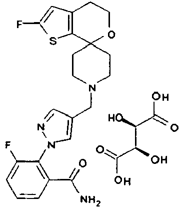 | 89 | 445 (M+1) |
| 15 | 1-[3-fluoro-2-[3-methyl-4-[(2,4,4-trifluorospiro[5H-thieno[2,3-c]pyran-7,4'-piperidine]-1'-yl)methyl]pyrazol-1-yl]phenyl]-*N,N*-dimethyl-methanamine (*L*)-Tartrate | 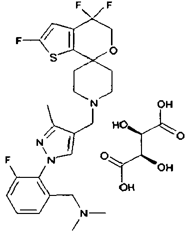 | 57 | 509 (M+1) |
| 16 | 1-[2-[4-[(2-chlorospiro[4,5-dihydrothieno[2,3-c]pyran-7,4'-piperidine]-1'-yl)methyl]-3-methyl-pyrazol-1-yl]-3-fluoro-phenyl]-*N,N-*dimethyl-methanamine (*L*)-Tartrate | 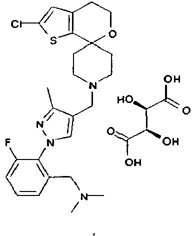 | 51 | 489 (M+1) |
| 17 | 2-chloro-4,4-difluoro-1'-[[1-[2-fluoro-6-(methoxymethyl)phenyl]-3-methyl-pyrazol-4-yl]methyl]spiro[5H-thieno[2,3-c]pyran-7,4'-piperidine] (*L*)-Tartrate | 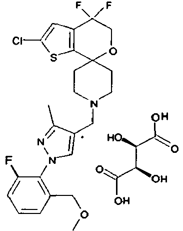 | 55 | 512 (M+1) |
| 18 | 3-fluoro-2-[3-methyl-4-[(2,4,4-trifluorospiro[5H-thieno[2,3-c]pyran-7,4'-piperidine]-1'-yl)methyl]pyrazol-1-yl]benzamide (*L*)-Tartrate | 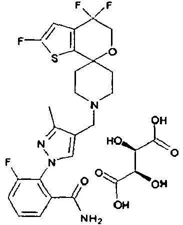 | 75 | 495 (M+1) |
| 19 | 2-[4-[(2-chloro-4,4-difluoro-spiro[5H-thieno[2,3-c]pyran-7,4'-piperidine]-1'-yl)methyl]pyrazol-1-yl]-3-fluoro-benzonitrile (*L*)-Tartrate | 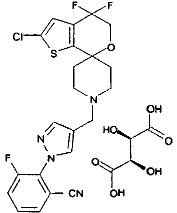 | 59 | 479 (M+1) |
| 20 | 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-benzamide (*L*)-Tartrate | 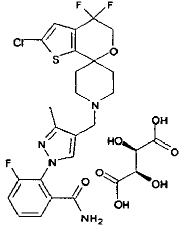 | 84 | 511 (M+1) |
| 21 | 3-chloro-2-[4-[(2-chloro-4,4-difluoro-spiro[5H-thieno[2,3-c]pyran-7,4'-piperidine]-1'-yl)methyl]-3-methyl-pyrazol-1-yl]benzamide (*L*)-Tartrate | 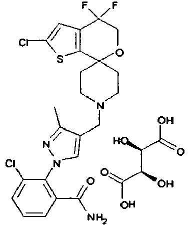 | 27 | 527 (M+1) |

| | | | | |
|---|---|---|---|---|
| ^{a} Reaction carried out using tetrahydrofun as solvent. | | | | |

### Example 22: 2'-Chloro-1-((1-(2,6-dimethylphenyl)-3-methyl-1H-pyrazol-4-yl)methyl)-4',4'-difluoro-4',5'-dihydrospiro[piperidine-4,7'-thieno[2,3-c]pyran]

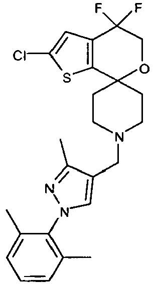

To a screw-cap test tube containing a mixture of 1-(2,6-dimethylphenyl)-3-methyl-1H-pyrazole-4-carbaldehyde (0.067 g, 0.31 mmol), 2-chloro-4,4-difluoro-spiro[5H-thieno[2,3-c]pyran-7,4'-piperidine] (0.088 mg, 0.31 mmol) and acetic acid (18 µL, 0.31 mmol) in 1,2-dichloroethane (1.1 mL) is added sodium triacetoxyborohydride (0.2 g, 0.94 mmol). The reaction tube is sealed and stirred at room temperature for 18 hr. with the aid of a magnetic stirrer. The mixture is diluted with methanol and purified using a 2 g SCX cartridge. The solvent is evaporated *in vacuo* and the resulting residue is purified by normal phase Isco chromatography eluting with hexane:ethanol (2-20% in ethanol) to give 0.099 g of the title compound. MS (m/z): 478 (M+1).

### Example 23: 2-Chloro-4,4-difluoro-1'-[[1-[2-fluoro-6-(1-methylimidazol-2-yl)phenyl]-3-methyl-pyrazol-4-yl]methyl]spiro[5H-thieno[2,3-c]pyran-7,4'-piperidine]

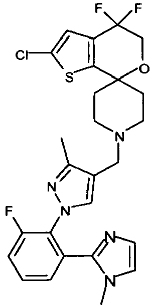

To a screw-cap test tube containing a mixture of 1-[2-fluoro-6-(1-methylimidazol-2-yl)phenyl]-3-methyl-pyrazole-4-carbaldehyde (288 mg, 1.01 mmol) (contaminated with the other pyrazole regioisomer in a ratio 75:25) and 2-chloro-4,4-difluoro-spiro[5H-thieno[2,3-c]pyran-7,4'-piperidine] (311.72 mg, 1.11 mmol) in 1,2-dichloroethane (3 mL) is stirred at room temperature for 1 hr. and then sodium triacetoxyborohydride (429.41 mg, 2.03 mmol) is added. The reaction tube is sealed and stirred at room temperature for 18 hr. with the aid of a magnetic stirrer. Then, the reaction is quenched by addition of sodium bicarbonate saturated solution and the compound is extracted with ethyl acetate. The organic layer is separated, dried over magnesium sulfate and the solvent removed under reduced pressure. The compound is purified by supercritical fluid chromatography using AD-H as stationary phase to provide 230 mg (41%) of the title compound as white solid. MS (m/z): 548 (M+1).

### Example 24: Methyl N-[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]-N-methyl-carbamate (L)-Tartrate

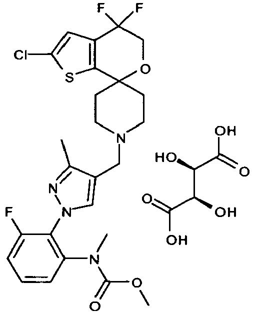

To a solution of 2-chloro-4,4-difluoro-spiro[5H-thieno[2,3-c]pyran-7,4'-piperidine] (210 mg, 0.75 mmol) in dichloromethane (3.00 mL), methyl *N*-[3-fluoro-2-(4-formyl-3-methyl-pyrazol-1-yl)phenyl]-*N*-methyl-carbamate (284.27 mg) (as major compound in a mixture of regioisomers in the pyrazole) is added. The mixture is stirred 10 min at room temperature. Then, sodium triacetoxyborohydride (331.5 mg) is added, and the reaction is stirred at room temperature overnight. The mixture is diluted with dichloromethane and quenched slowly with sodium bicarbonate (saturated solution). The organic phase is then extracted with more dichloromethane, decanted, dried over magnesium sulfate and solvent evaporated under reduced pressure. The residue is purified by normal phase Isco chromatography using as eluent dichloromethane and methanol to give 160 mg of methyl *N*-[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]-*N*-methyl-carbamate. MS (m/z): 555 (M+1).

The tartrate salt is essentially prepared as described in Example 1. MS (m/z): 555 (M+1).

### Example 25: N-[[2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-phenyl]methyl]cyclopropanamine (L)-Tartrate

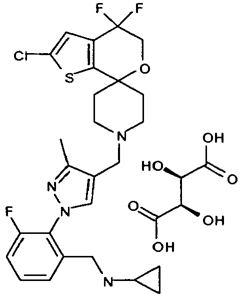

A solution of 2-chloro-4,4-difluoro-spiro[5H-thieno[2,3-c]pyran-7,4'-piperidine] (0.220 g, 0.788 mmol) and cyclopropyl-[3-fluoro-2-(4-formyl-3-methyl-pyrazol-1-yl)-benzyl]-carbamic acid tert-butyl ester (0.327 g, 0. 876 mmol) in tetrahydrofuran (0.3 mL) is stirred at room temperature for 1 hr. and then sodium triacetoxyborohydride (0.371g, 1.75 mmol) is added. The mixture is stirred overnight. The solvent is evaporated *in vacuo* and the residue is dissolved in 4 N hydrochloric acid in iso-propyl alcohol and stirred at room temperature for 2 hr. The solvent is evaporated and the residue is diluted in methanol and charged in an SCX cartridge. The 2 N ammonia in methanol fraction is collected and evaporated. The residue is purified by reverse phase HPLC to give 0.142 g of *N*-[[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]methyl]cyclopropanamine. MS (m/z): 537 (M+1).

The tartrate salt is essentially prepared as described in Example 1. MS (m/z): 537 (M+1).

### Example 26: 2-Chloro-4,4-difluoro-1'-[[1-(2-fluorophenyl)-3-methyl-pyrazol-4-yl]methyl]spiro[5H-thieno[2,3-c]pyran-7,4'-piperidine] (L)-Tartrate

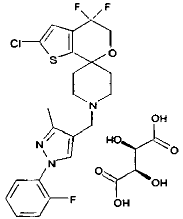

To a screw-cap test tube is added copper (I) iodide (6.1 mg, 0.32 mmol), 2-chloro-4,4-difluoro-1'-[(3-methyl-1H-pyrazol-4-yl)methyl]spiro[5H-thieno[2,3-c]pyran-7,4'-piperidine] (120 mg, 0.32 mmol), potassium carbonate (94 mg, 0.67 mmol), 1 mL of toluene (previously bubbled with nitrogen for 20 minutes) and a stir bar. The reaction mixture is bubbled with nitrogen for additional 10 min and then 1-fluoro-2-iodobencene (106 mg, 0.48 mmol), and *trans*-N,N'-dimethylcyclohexane-1,2-diamine (0.01 mL, 0.64 mmol) are added. The reaction tube is quickly sealed (caution: build-up of pressure possible; use a safety shield) and immersed in a preheated oil bath at 115°C for 16 hr. The sample is cooled down to room temperature, diluted with ethyl acetate and filtered through celite. The solvent is evaporated *in vacuo*. The residue is purified by normal phase Isco chromatography using hexane/ethyl acetate (10-40% in ethyl acetate) to yield 103.9 mg of the title compound. MS (m/z): 468 (M+1).

The tartrate salt is essentially prepared as described in Example 1. MS (m/z): 468 (M+1).

The compounds of Example 27-29 are prepared essentially as described in Example 26 from the corresponding phenyl halide.

| Ex. No. | Chemical name | Structure | Yield (%) | Physical data: MS (m/z) |
|---|---|---|---|---|
| 27 | 2-chloro-4,4-difluoro-1'-[[1-[2-(methoxymethyl)phenyl]-3-methyl-pyrazol-4-yl]methyl]spiro[5H-thieno[2,3-c]pyran-7,4'-piperidine] (*L*)-Tartrate | 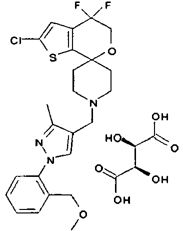 | 28 | 480 (M+1) |
| 28 | 2-chloro-4,4-difluoro-1'-[[1-(2-isopropylphenyl)-3-methyl-pyrazol-4-yl]methyl]spiro[5H-thieno[2,3-c]pyran-7,4'-piperidine] (*L*)-Tartrate | 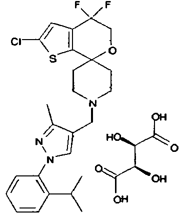 | 10 | 493 (M+1) |
| 29 | 2-chloro-1'-[[1-(2,6-difluorophenyl)pyrazol-4-yl]methyl]spiro[4,5-dihydrothieno[2,3-c]pyran-7,4'-piperidine] (*L*)-Tartrate | 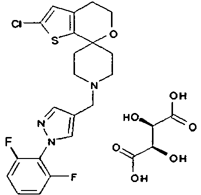 | 22 | 436 (M+1) |

### Example 30: 2'-Chloro-4',4'-difluoro-1-((1-(2-tolyl)-3-methyl-1H-pyrazol-4-yl)methyl)-4',5'-dihydrospiro[piperidine-4,7'-thieno[2,3-c]pyran]

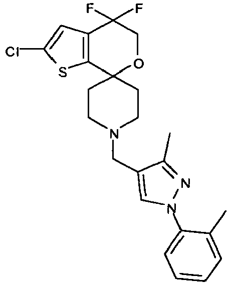

To a screw-cap test tube are added copper(I) iodide (23.74 mg, 0.125 mmol), 2-chloro-4,4-difluoro-1'-[(3-methyl-1H-pyrazol-4-yl)methyl]spiro[5H-thieno[2,3-c]pyran-7,4'-piperidine] (233 mg, 0.623 mmol), potassium carbonate (182.71 mg, 1.31 mmol), toluene (2 mL) (previously bubbled with nitrogen for 20 min) and a stir bar. The reaction mixture is bubbled with nitrogen for 20 minutes and then 2-iodo-toluene (272 mg, 1.25 mmol) and *trans*-N,N'-dimethylcyclohexane-1,2-diamine (39.31 µL, 0.25 mmol) are added. The reaction tube is quickly sealed (caution: build-up of pressure possible; use a safety shield) and immersed in a preheated oil bath at 110°C for 24 hours with the aid of a magnetic stirrer. Then, the mixture is poured on SCX column (25g) and eluted with methanol and then 2N solution of ammonia in methanol. The basic fraction is concentrated and the resulting residue is purified by normal phase Isco chromatography eluting with cichloromethane:ethanol (gradient from 5 to 20% in ethanol) to give the title compound in a 16% yield. MS (m/z): 464 (M+1).

The tartrate salt is essentially prepared as described in Example 1. MS (m/z): 464 (M+1).

### Example 31: 2'-Chloro-1-((1-(2-fluro-6-methoxyphenyl)-3-methyl-1H-pyrazol-4-yl)methyl)-4',4'-difluoro-4',5'-dihydrospiro[piperidine-4,7'-thieno[2,3-c]pyran]

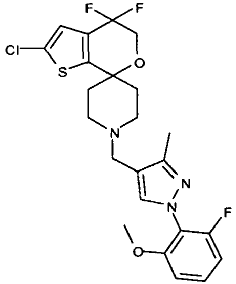

To a screw-cap test tube containing 1-fluoro-2-iodo-3-methoxybenzene (607 mg, 2.41 mmol) in dry dimethylformamide (3.2 mL) are added under nitrogen 4-((2'-chloro-4',4'-difluoro-4',5'-dihydrospiro[piperidine-4,7'-thieno[2,3-c]pyran]-1-yl)methyl)-3-methyl-1H-pyrazole (600 mg 1.6 mmol), copper(I) oxide (23 mg, 160 µmoles), (R,R)-(-)-N,N'-dimethyl-1,2-cyclohexanediamine (68 mg, 480 µmoles) and cesium carbonate (1040 mg, 3.2 mmol). The reaction tube is quickly sealed (caution: build-up of pressure possible; use a safety shield) and stirred in a preheated oil bath at 110°C for 16 hr. with the aid of a magnetic stirrer. The mixture is diluted with water and extracted with ethyl acetate. The organic layer is separated, dried over magnesium sulfate, filtered and the solvent evaporated *in vacuo.* The resulting residue is purified by silica gel using normal phase Isco chromatography eluting with hexane:ethanol (gradient from 2 to 15% in ethanol) to give 17% yield of the title compound. MS (m/z): 498 (M+1).

### Example 32: 1-[2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-phenyl]-N-methyl-methanamine (L)-Tartrate

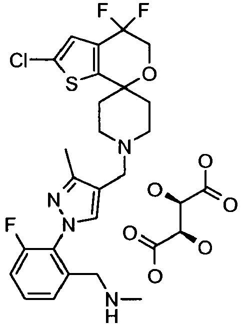

### 1. 2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-benzaldehyde

Manganese (IV) oxide (13.89 g, 140.57 mmol) is added to a solution of [2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]methanol (28.00 g, 56.23 mmol) in dichloromethane (224 mL) at room temperature and the resulting suspension is stirred at reflux for 2.5 hr. Additional manganese (IV) oxide (33.33 g, 337.37 mmol) is added and the mixture is stirred at reflux for 4h and at room temperature for 15 hr. More manganese (IV) oxide (8.33 g, 84.34 mmol) is added and stirring is continued for 7 hr. at room temperature. Reaction mixture is left stand for 1 hr. without stirring. Supernatant is decanted and filtered through a pad of celite eluting with dichloromethane. Filtrate is concentrated to give 27 g of 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-benzaldehyde. MS (m/z): 496 (M+1).

### 2. 1-[2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-phenyl]-N-methyl-methanamine (L)-Tartrate

40% Monomethylamine in water (3.25 mL, 37.71 mmol) is added to a solution of 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-benzaldehyde (17.00 g, 34.28 mmol) in ethanol (170 mL) at 0°C (ice/water bath). The resulting mixture is stirred at room temperature for 15 hr. It is cooled with ice/water bath and sodium borohydride (0.778 g, 20.57 mmol) is added and the mixture is stirred at room temperature for 3 hr. 5% Hydrochloric acid is added droppwise at 0°C untill no gas evolution is observed (pH=6, around 20 mL) and mixture is concentrated to 1/4 of volume. Saturated aqueous solution of sodium bicarbonate is added (100 mL) and the resulting suspension is extracted with ethyl acetate (3 x 100 mL). Combined organic layers are dried over sodium sulfate and concentrated to afford a crude that is purified by silica gel chromatography eluting with ammonium hydroxide in methanol/dichloromethane mixtures to give 14 g of the free base of the title compound. MS (m/z): 511 (M+1).

The tartrate salt is essentially prepared as described in Example 1. MS (m/z): 511 (M+1).

### Example 33: 2-Chloro-4,4-difluoro-1'-[[1-[2-fluoro-6-(pyrrolidin-1-ylmethyl)phenyl]-3-methyl-pyrazol-4-yl]methyl]spiro[5H-thieno[2,3-c]pyran-7,4'-piperidine] (L)-Tartrate

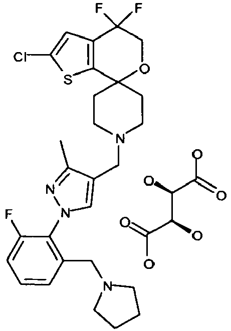

The free base of the title compound is essentially prepared as described in Preparation 7 using 2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-benzaldehyde and pyrrolidine in a 32% yield. MS (m/z): 551 (M+1).

The tartrate salt is essentially prepared as described in Example 1. MS (m/z): 551 (M+1).

The compounds of Example 34-35 are prepared essentially as described in Example 33 using the corresponding amine.

| Ex. No. | Chemical name | Structure | Yield (%) | Physical data: MS (m/z) |
|---|---|---|---|---|
| 34 | *N*-[[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]methyl]-2-methyl-propan-2-amine (*L*)-Tartrate | 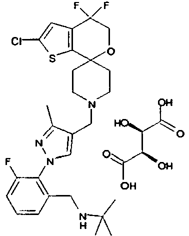 | 17 | 517 (M+1) |
| 35 | 2-chloro-1'-[[1-[2-[(3,3-difluoroazetidin-1-yl)methyl]-6-fluoro-phenyl]-3-methyl-pyrazol-4-yl]methyl]-4,4-difluoro-spiro[5H-thieno[2,3-c]pyran-7,4'-piperidine] (*L*)-Tartrate | 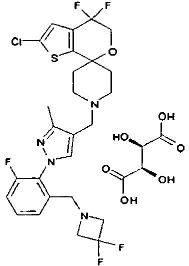 | 36 | 573 (M+1) |

### Example 36: N-(2-(4-((2'-Chloro-4',4'-difluoro-4',5'-dihydrospiro[piperidine-4,7'-thieno[2,3-c]pyran]-1 yl)methyl)-3-methyl-1H-pyrazol-1-yl)-3-fluorobenzylidene)-2-methoxyethanamine

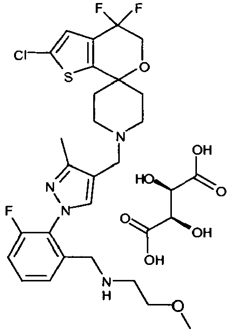

A solution of 2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-fluorobenzaldehyde (0.220 g, 0.44 mmol) and 2-methoxyethyl amine (0.058 mL, 0.67 mmol) in 2.2 mL of anhydrous dichloromethane is stirred overnight with 4 A molecular sieves. The mixture is filtrated and solvent evaporated *in vacuo* to give the corresponding imine in quantitative yield. MS (m/z): 553 (M+1).

Sodium borohydride (0.050 g, 1.32 mmol) and 2 drops of methanol are added to a solution of *N*-(2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-fluorobenzylidene)-2-methoxyethanamine (0.237 g, 0.44 mmol) in 2.2 mL of 1,2-dichloroethane and the mixture is stirred for 24 hr. 1N sodium hydroxide is added over the solution to quench the reaction and it is extracted with ethyl acetate, washed with water and brine, and dried over magnesium sulfate. The residue obtained after filtration and evaporation of solvent is purified using a 2g SCX cartridge. The resulting product is purified by normal phase Isco chromatography eluting with ethanol and 15% solution of ammonium hydroxide (7N in methanol) in ethanol (50-90% gradient of the basic eluent) to yield 0.075 g of the free base of the title compound. MS (m/z): 555 (M+1).

The tartrate salt is essentially prepared as described in Example 1. MS (m/z): 555 (M+1).

### Example 37: 2-(2-(4-((2'-Chloro-4',4'-difluoro-4',5'-dihydrospiro[piperidine-4,7'-thieno[2,3-c]pyran]-1-yl)methyl)-3-methyl-1H-pyrazol-1-yl)-3-fluorobenzylamino)ethanol

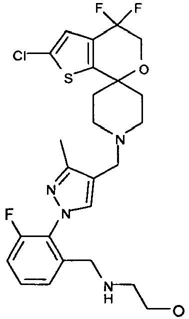

Sodium triacetoxyborohydride (0.280 g, 1.32mmol) is added to a solution of 2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-fluorobenzaldehyde (0.163 g, 0.33 mmol) and 2-aminoethanol (0.022 mL, 0.36 mmol) in 1.8 mL of 1,2-dichloroethane. Mixture is stirred overnight. Saturated aqueous solution of sodium bicarbonate is added and mixture is extracted in ethyl acetate, washed with water and brine, and dried over magnesium sulfate. Residue after filtration and evaporation is purified using a 2g SCX cartridge. The resulting product is further purified by normal phase Isco chromatography eluting with hexane:ethanol (50-90% in ethanol) yielding 0.070 g of the title compound. MS (m/z): 541 (M+1).

### Example 38: (2-(4-((2'-Chloro-4',4'-difluoro-4',5'-dihydrospiro[piperidine-4,7'-thieno[2,3-c]pyran]-1-yl)methyl)-3-methyl-1H-pyrazol-1-yl)-3-fluorophenyl)methanamine (L)-Tartrate

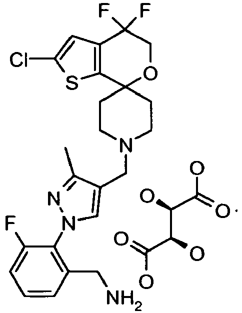

### 1. 2-(2-(4-((2'-Chloro-4',4'-difluoro-4',5'-dihydrospiro[piperidine-4,7'-thieno[2,3-c]pyran]-1-yl)methyl)-3-methyl-1H-pyrazol-1-yl)-3-fluorobenzyl)-isoindole-1,3-dione

Diisopropyl azodicarboxylate (0.105 mL, 0.54 mmol) is added to a solution of (2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-fluorophenyl)methanol (0.180 g, 0.36 mmol), phtalimide (0.079 mg, 0.54 mmol) and triphenylphosphine (0.142 g, 0.54 mmol) in 3 mL toluene at 0°C. The mixture is stirred at room temperature overnight. The solvent is removed and the residue is purified first using a 2 g SCX cartridge and after evaporation of the 2 N ammonia in methanol fraction by normal phase Isco chromatography eluting with hexane/ethanol (3%-30%) to give 0.204 g of 2-(2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-fluorobenzyl)-isoindole-1,3-dione. MS (m/z): 627 (M+1).

### 2. (2-(4-((2'-Chloro-4',4'-difluoro-4',5'-dihydrospiro[piperidine-4,7'-thieno[2,3-c]pyran]-1-yl)methyl)-3-methyl-1H-pyrazol-1-yl)-3-fluorophenyl)methanamine (L)-Tartrate

To a flask containing 2-(2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-fluorobenzyl)-isoindole-1,3-dione (0.204 g, 0.33 mmol) and 24 µl of water in ethanol (2.5 mL) is added hydrazine monohydrate (0.04 g, 0.79 mmol). Reaction mixture is refluxed for 2.5 hr. and then diluted with methanol and purified using a 2g SCX cartridge. After evaporation of the 2N ammonia in methanol fraction, the resulting product is purified by normal phase Isco chromatography eluting with ethanol and a 15% solution of ammonium hydroxide (7N in methanol) in ethanol (gradient of 25-90% of the basic eluent) yielding 75 mg of (2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-fluorophenyl)methanamine. MS (m/z): 497 (M+1).

The tartrate salt is essentially prepared as described in Example 1. MS (m/z): 497 (M+1).

### Example 39: (2-(4-((2'-Chloro-4',4'-difluoro-4',5'-dihydrospiro[piperidine-4,7'-thieno[2,3-c]pyran]-1-yl)methyl)-3-methyl-1H-pyrazol-1-yl)-3-cyclopropylphenyl)methanol

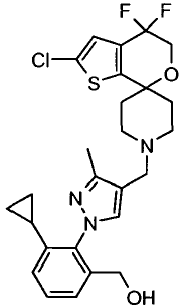

### 1. Methyl 2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-cyclopropylbenzoate

To a screw-cap test tube containing a mixture of methyl 3-cyclopropyl-2-(4-formyl-3-methyl-1H-pyrazol-1-yl)benzoate (0.059 g, 0.21 mmol), 2'-chloro-4',4'-difluoro-4',5'-dihydrospiro[piperidine-4,7'-thieno[2,3-c]pyrane (0.058 g, 0.13 mmol) and acetic acid (12 µL, 0.21 mmol) in 1,2-dichloroethane (0.73 mL) is added sodium triacetoxyborohydride (0.066 g, 0.31 mmol). The reaction tube is sealed and stirred at room temperature for 18 hr. with the aid of a magnetic stirrer. The mixture is diluted with methanol and purified using a 2g SCX cartridge and the solvent evaporated *in vacuo* to give 0.113 g of methyl 2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-cyclopropylbenzoate. MS (m/z): 548 (M+1)

### 2. (2-(4-((2'-Chloro-4',4'-difluoro-4',5'-dihydrospiro[piperidine-4,7'-thieno[2,3-c]pyran]-1-yl)methyl)-3-methyl-1H-pyrazol-1-yl)-3-cyclyopropylphenyl)methanol

To a screw-cap test tube containing a suspension of lithium aluminium hydride (9 mg, 0.23 mmol) in tetrahydrofuran (0.8 mL) is added, under nitrogen at 0°C, a solution of methyl 2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-cyclopropylbenzoate (0.113 g, 0.21 mmol) in anhydrous tetrahydrofuran (0.4 mL). The reaction tube is sealed and stirred at 0°C for 30 min with the aid of a magnetic stirrer. The mixture is diluted with water (0.4 mL), stirred for 30 min and diluted with methanol. The product is purified using a 2g SCX cartridge and after evaporation of the basic fraction, the resulting residue is purified by normal phase Isco chromatography eluting with hexane:ethanol (2-15% in ethanol) to give 0.070 mg of the title compound. MS (m/z): 520 (M+1).

### Example 40: 1-(2-(4-((2'-Chloro-4',4'-difluoro-4',5'-dihydrospiro[piperidine-4,7'-thieno[2,3-c]pyran]-1-yl)methyl)-3-methyl-1H-pyrazol-1-yl)-3-fluorophenyl)ethanone

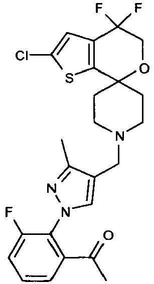

To a screw-cap test tube containing 4-((2'-chloro-4',4'-difluoro-4',5'-dihydrospiro[piperidine-4,7'-thieno[2,3-c]pyran]-1-yl)methyl)-3-methyl-1H-pyrazole (0.271 g, 0.72 mmol) in dimethylformamide (2.2 mL) are added 1-(2,3-difluorophenyl)ethanone (0.124 g, 0.8 mmol) and potassium carbonate (0.150 g, 1.1 mmol). The reaction tube is quickly sealed (caution: build-up of pressure possible; use a safety shield) and stirred in a preheated oil bath at 110°C for 18 hr. with the aid of a magnetic stirrer. The mixture is diluted with methanol and purified using a 2g SCX cartridge and the solvent is evaporated *in vacuo.* The resulting residue is purified by normal phase Isco chromatography eluting with dichloromethane/methanol (2-20% in methanol) to give 0.075 g of the title compound. MS (m/z): 510 (M+1).

### Example 41: 2-Chloro-1'-[[1-(2-chlorophenyl)-3-methyl-pyrazol-4-yl]methyl]-4,4-difluoro-spiro[5H-thieno[2,3-c]pyran-7,4'-piperidine] (L)-Tartrate

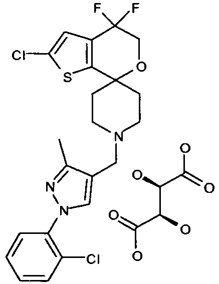

A screw-cap test tube containing molecular sieves (4A) and a solution of 2-chloro-4,4-difluoro-1'-[(3-methyl-1H-pyrazol-4-yl)methyl]spiro[5H-thieno[2,3-c]pyran-7,4'-piperidine] (0.250 g, 0.675 mmol), 2-chlorobenzeneboronic acid (0.212 g, 1.35 mmol), copper(II) acetate (0.185 g, 1.01 mmol), pyridine (0.083 mL, 1.35mmol) in anhydrous dichloromethane (1.3 mL) is shaked for 48 hr. Reaction crude is diluted with methanol and first purified using a 5g SCX cartridge. Resulting product is further purified by normal phase Isco chromatography eluting with dichloromethane/ethanol (5-30% in ethanol) to give 0.067 g of the title compound. MS (m/z): 535 (M+1).

The tartrate salt is essentially prepared as described in Example 1. MS (m/z): 535 (M+1).

### Example 42: 1-[2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-phenyl]cyclopropanamine (L)-Tartrate

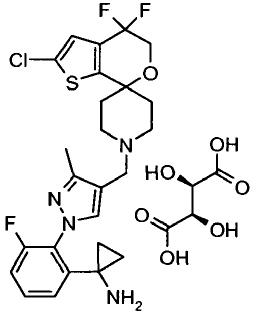

### 1. 2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-benzonitrile

To a suspension of 2-chloro-4,4-difluoro-spiro[5H-thieno[2,3-c]pyran-7,4'-piperidine] hydrochloride (1.3 g, 4.11 mmol) in 1,2-dichloroethane (20.5 mL) is added triethylamine (0.75 mL, 5.3 mmol) and 3-fluoro-2-(4-formyl-3-methyl-pyrazol-1-yl)-benzonitrile (as major compound in a mixture of regioisomers) (1.13 g, 4.93 mmol). The mixture is stirred at room temperature for 30 min. Then, sodium triacetoxyborohydride (1.82 g, 8.22 mmol) is added. The mixture is stirred at room temperature overnight. After that time, solvent is evaporated and the residue is diluted with methanol and purified using a 50 g SCX cartridge and then by normal phase Isco chromatography eluting with dichloromethane and methanol to give 1.6 g of a crude material containing a mixture of regioisomers (80:20 aprox). This residue is further purified by reverse phase HPLC to give 927 mg of 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-benzonitrile as the major regioisomer. MS (m/z): 493 (M+1).

### 2. 1-[2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-phenyl]cyclopropanamine (L)-Tartrate

To a suspension of 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-benzonitrile (0.530 g, 1.08 mmol) and titanium tetra(isopropoxide) (0.350 mL, 1.19 mol) in diethyl ether (10.75 mL) under nitrogen, cooled to -70°C, 3.0 M ethylmagnesium bromide in ethyl ether (0.789 mL, 2.42 mmol) is added. Cooling bath is removed and the mixture is stirred at room temperature for 1 hr. (after removing the cooling bath a precipitate appeared and the mixture became a black solution along the time). Boron trifluoride etherate (0.272 mL, 2.16 mmol) is added and stirred for 1 hr. at room temperature (a precipitate appeared again and it turned reddish progressively). After that time, 1 M hydrochloric acid (3.5 mL) is added followed by *tert*-butyl methyl ether (18 mL). 10% Sodium hydroxide (15 mL) is added to the mixture and it is extracted with *tert*-butyl methyl ether. Organic layer is decanted and solvent evaporated. Crude is purified by normal phase Isco chromatography eluting with dichloromethane/methanol mixtures to give 160 mg of 1-[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]cyclopropanamine. MS (m/z): 523 (M+1).

The tartrate salt is essentially prepared as described in Example 1. MS (m/z): 523 (M+1).

### Example 43: 1-[2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-phenyl]-N,N-dimethyl-cyclopropanamine (L)-Tartrate

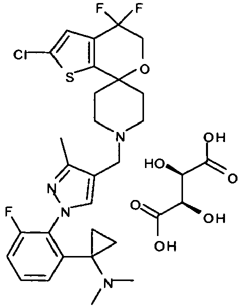

To a solution of 1-[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]cyclopropanamine (100 mg, 0.19 mmol) in tetrahydrofuran (1.91 mL) is added formaldehyde (37% in water) (17.24 µL) and the mixture is stirred at room temperature for 10 min. Then sodium triacetoxyborohydride (81.05 mg) is added and the reaction mixture stirred at room temperature overnight. The reaction is not completed and more of formaldehyde (2 eq), and sodium triacetoxyborohydride (2 eq) are added, and the stirring is continued at room temperature for 2 hr. Then, the solvent is evaporated and the residue is purified using a 5 g SCX cartridge. After evaporation of the 2 N ammonia in methanol fraction the residue is further purified by normal phase Isco chromatography using dichloromethane/methanol as eluent to give 1-[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]-*N,N*-dimethyl-cyclopropanamine in a 68% yield. MS (m/z):551 (M+1).

The tartrate salt is essentially prepared as described in Example 1. MS (m/z): 551 (M+1).

### Example 44: N-[[2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-phenyl]methyl]-N-methylacetamide (L)-Tartrate

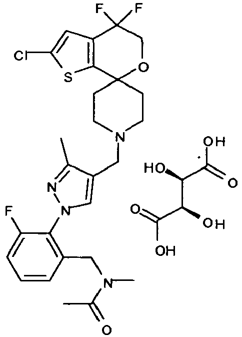

Acetic acid anhydride (0.043 mL, 0.452 mmol) is added to a solution of 1-[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]-*N*-methyl-methanamine (0.210 g, 0.411 mmol) in dichloromethane (4 mL) at 0°C. After 1 hr. , saturated aqueous solution of sodium bicarbonate is added to the reaction and the crude is extracted with dichloromethane. The organic layer was separated, dried over magnesium sulfate and the solvent evaporated *in vacuo* to afford 0.21 g of *N*-[[2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-phenyl]methyl]-*N*-methyl-acetamide. MS (m/z): 533 (M+1)

The tartrate salt is essentially prepared as described in Example 1. MS (m/z): 533 (M+1).

### Example 45: 1-[2-[4-[(2-Chloro-4,4-difluoro-spiro[5H-thieno[2,3-c]pyran-7,4'-piperidine]-1'-yl)methyl]-3-methyl-pyrazol-1-yl]-3-methoxy-phenyl]-N-methyl-methanamine (L)-Tartrate

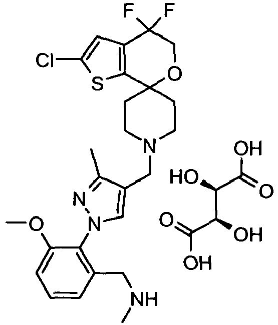

To a solution of 2-chloro-4,4-difluoro-1'-[(3-methyl-1H-pyrazol-4-yl)methyl]spiro[5H-thieno[2,3-c]pyran-7,4'-piperidine] (0.125 g, 0.334 mmol) in dimethylformamide (1 mL) are added 2-fluoro-3-methoxybenzaldehyde (0.501 mmol, 0.080 mg) and cesium carbonate (0.142 g, 0.435 mmol). The mixture is heated at 110°C for 24 hr. Water is added and the organic layer is extracted with ethyl acetate, washed with brine, dried over magnesium sulfate and filtered. The solvent is evaporated under reduced pressure. A 5 g silica cartridge is used to purify the residue using hexane/ethyl acetate 1:1 as eluent until the major impurity is came up followed by ethyl acetate to obtain 0.065 g of impure aldehyde. This material is dissolved in tetrahydrofuran (4 mL) and monomethylamine (0.640 mL, 1.28 mmol) is added. The mixture is stirred at room temperature for 1 hr. and then, sodium triacetoxyborohydride (0.1 g) and a few drops of acetic acid are added. The reaction mixture is stirred at room temperature for 10 hr. Water and saturated aqueous solution of sodium bicarbonate are added. The reaction mixture is extracted with ethyl acetate. The organic layer is dried over magnesium sulfate, filtered and the solvent is removed under reduced pressure. This compound is purified using basic reverse phase HPLC with no success and later using chiral chromatography to yield 18.5 mg of the title compound as free base. MS (m/z): 523 (M+1) (Chiral purification conditions: stationary phase: Chiralcel OD; mobile phase: n-hexane-0.2%dimethyll ethyl amine/ethanol; column size: 10µm,20*250; elution mode: isocratic 85/15; llow rate: 12 (mL/min); UV detection: 254.16 (nm); loading: 27mg)

The tartrate salt is essentially prepared as described in Example 1. MS (m/z): 523 (M+1).

### Example 46: 2-Chloro-4,4-difluoro-1'-[[1-(2-fluoro-6-imidazol-1-yl-phenyl)-3-methyl-pyrazol-4-yl]methyl]spiro[5H-thieno[2,3-c]pyran-7,4'-piperidine] (L)-Tartrate

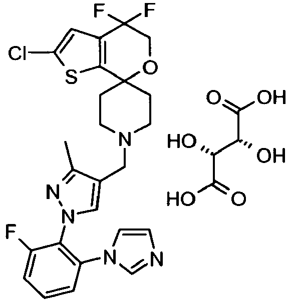

### 1. 2-Chloro-4,4-difluoro-1'-[[1-(2-fluoro-6-nitro-phenyl)-3-methyl-pyrazol-4-yl]methyl]spiro[5H-thieno[2,3-c]pyran-7,4'-piperidine]

A solution of 2-chloro-4,4-difluoro-spiro[5H-thieno[2,3-c]pyran-7,4'-piperidine] (2.1 g, 7.5 mmol) and 1-(2-fluoro-6-nitro-phenyl)-3-methyl-pyrazole-4-carbaldehyde (3 g) (as major compound in a mixture of regioisomers in the pyrazole) in dichloromethane (47 mL) is stirred at room temperature for 30 minutes. Then, sodium triacetoxy borohydride (3.94g) is added and the mixture is stirred overnight at that temperature. The reaction mixture is quenched carefully with sodium bicarbonate (saturated solution) and extracted with dichloromethane. Organic layer is washed with brine, decanted and dried over sodium sulfate. Solvent is evaporated and the residue is purified by normal phase Isco chromatography using as eluant dichloromethane and isopropanol to give 2.28 g of 2-chloro-4,4-difluoro-1'-[[1-(2-fluoro-6-nitro-phenyl)-3-methyl-pyrazol-4-yl]methyl]spiro[5H-thieno[2,3-c]pyran-7,4'-piperidine] (the compound is contaminated with the other pyrazole regioisomer in a ratio 80:20). MS (m/z): 513 (M+1).

### 2. 2-[4-[(2-Chloro-4,4-difluoro-spiro[5H-thieno[2,3-c]pyran-7,4'-piperidine]-1'-yl)methyl]-3-methyl-pyrazol-1-yl]-3-fluoro-aniline

To a solution of 2-chloro-4,4-difluoro-1'-[[1-(2-fluoro-6-nitro-phenyl)-3-methyl-pyrazol-4-yl]methyl]spiro[5H-thieno[2,3-c]pyran-7,4'-piperidine] (2.28g, 4.46 mmol) and iron (2.5g) in ethanol (11mL) and water (11mL), a few drops of acetic acid is added. The reaction mixture is stirred at 90°C for 70 min. The mixture is then filtered over celite and concentrated under vacuum. Remained solution is basified with sodium bicarbonate (saturated solution) and extracted with dichloromethane. Organic layer is decanted, dried over sodium sulfate and solvent evaporated to obtain 1.8 g of 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-aniline that is used in next step without further purification (the compound is contaminated with the other pyrazole regioisomer in a ratio 80:20). MS (m/z): 483 (M+1).

### 3. 2-Chloro-4,4-difluoro-1'-[[1-(2-fluoro-6-imidazol-1-yl-phenyl)-3-methyl-pyrazol-4-yl]methyl]spiro[5H-thieno[2,3-c]pyran-7,4'-piperidine](L)-Tartrate

A mixture of 0.5 mL of acetic acid, formaldehyde (37.81 µL), and ethanedial (59.61 µL), is heated at 70°C. A solution of 0.5 mL of acetic acid, ammonium acetate (29.74 mg) and 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-aniline (250 mg, 0.52 mmol) is added drop wise to the flask over 15 min. The solution is continuously stirred at 70°C overnight. The reaction mixture is cooled to room temperature and poured slowly and carefully over 15 mL of a solution of sodium bicarbonate (90 mg/mL). The desired compound is then extracted with ethyl acetate. Organic layer is decanted, dried over magnesium sulfate and solvent evaporated. Crude is purified by normal phase Isco chromatography using as eluent dichloromethane/methanol mixtures from 98/2 to 90/10. Further purification by RP HPLC is needed to yield 89.7 mg of the title compound as free base. MS (m/z): 534 (M+1).

The tartrate salt is essentially prepared as described in Example 1. MS (m/z): 534 (M+1).

### Example 47: 2-Chloro-4,4-difluoro-1'-[[1-[2-fluoro-6-(2-methylimidazol-1-yl)phenyl]-3-methyl-pyrazol-4-yl]methyl]spiro[5H-thieno[2,3-c]pyran-7,4'-piperidine] (L)-Tartrate

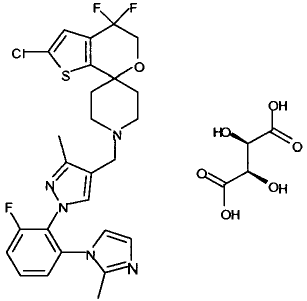

The title compound is prepared essentially as described in the third step of the preparation of Example 46 using 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-aniline(350.0 mg, 0.73 mmol) and acetaldehyde (0.163 mL, 2.90 mmol). The residue is purified by reverse phase HPLC to give 95.4 mg of desired compound, but further purification by normal phase HPLC is needed to yield 32 mg of the desired pure free base in 8% yield. The tartrate salt is essentially prepared as described in Example 1. MS (m/z): 548 (M+1).

### Example 48: 2-Chloro-4,4-difluoro-1'-[[1-[2-fluoro-6-(1H-1,2,4-triazol-3-yl)phenyl]-3-methyl-pyrazol-4-yl]methyl]spiro[5H-thieno[2,3-c]pyran-7,4'-piperidine]

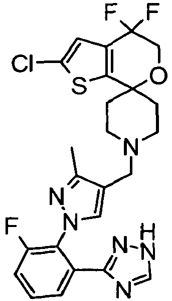

The mixture of 2-[4-[(2-chloro-4,4-difluoro-spiro[5H-thieno[2,3-c]pyran-7,4'-piperidine]-1'-yl)methyl]-3-methyl-pyrazol-1-yl]-3-fluoro-benzamide (455 mg) and methanamine, 1,1-dimethoxy-*N*,*N*-dimethyl- (3.12 mL, 23.40 mmol) is heated to 120°C for 2 hr. The reaction is cooled to room temperature and the solvent removed under reduced pressure. The residue is dissolved in acetic acid (2.68 mL) and hydrazine monohydrate (43.49 µl) is added. The resultant solution is heated to 90°C for 2 hr. The reaction is cooled to room temperature. Acetic acid is then removed under reduced pressure and the residue is diluted with ethyl acetate and washed with saturated solution of sodium bicarbonate. The organic phase is evaporated under vacuo. The crude mixture is purified by flash chromatography on silica gel chromatography using dichloromethane/2N ammonia in methanol mixtures to give 163 mg of the title compound as white solid. MS (m/z): 465 (M+1)

### Example 49: [4-[(2-Chloro-4,4-difluoro-spiro[5H-thieno[2,3-c]pyran-7,4'-piperidine]-1'-yl)methyl]-1-(2-fluorophenyl)pyrazol-3-yl]methanol

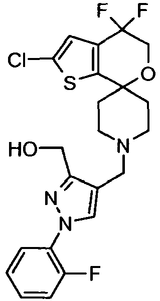

### 1. Ethyl 4-[(2-chloro-4,4-difluoro-spiro[5H-thieno[2,3-c]pyran-7,4'-piperidine]-1'-yl)methyl]-1-(2-fluorophenyl)pyrazole-3-carboxylate

To a solution of 2'-chloro-4',4'-difluoro-4',5'-dihydrospiro[piperidine-4,7'-thieno[2,3-c]pyran] hydrochloride (0.8 g, 2.53 mmol) and ethyl 1-(2-fluorophenyl)-4-formyl-pyrazole-3-carboxylate (0.79 g, 3.04 mmol) in 1,2-dichloroethane (25 mL) is added *N-*methyl morpholine (0.83 mL, 7.59 mmol) and molecular sieves (0.20 g). The reaction mixture is stirred at room temperature for 15 minutes. Sodium triacetoxyborohydride (1.61 g, 7.59 mmol) is added and stirred at room temperature for 16 hours. After completion, the reaction mixture is filtered through celite and partitioned between dichloromethane (50 mL) and water (25 mL). The aqueous phase is extracted with dichloromethane (2 x 25 mL) and the combined organic extracts are dried over sodium sulfate and concentrated *in vacuo*. The crude mixture is purified by column chromatography over silica gel eluting with hexane/ethyl acetate (80:20) to yield 1.1 g (84%) of ethyl 4-[(2-chloro-4,4-difluoro-spiro[5H-thieno[2,3-c]pyran-7,4'-piperidine]-1'-yl)methyl]-1-(2-fluorophenyl)pyrazole-3-carboxylate. MS (m/z): 526 (M+1).

### 2. [4-[(2-Chloro-4,4-difluoro-spiro[5H-thieno[2,3-c]pyran-7,4'-piperidine]-1'-yl)methyl]-1-(2-fluorophenyl)pyrazol-3-yl]methanol

To a solution of ethyl 4-[(2-chloro-4,4-difluoro-spiro[5H-thieno[2,3-c]pyran-7,4'-piperidine]-1'-yl)methyl]-1-(2-fluorophenyl)pyrazole-3-carboxylate (0.9 g, 1.71 mmol) in tetrahydrofuran (10 mL) and ethanol (10 mL) is added lithium borohydride (3.40 mL, 2.0 M solution in tetrahydrofuran, 6.85 mmol) at 0 °C and stirred at room temperature for 16 hours. After completion, the reaction mixture was quenched with water (10 mL) and extracted with ethyl acetate (2 x 25 mL). The combined organic extracts are dried over sodium sulfate and concentrated *in vacuo.* The crude mixture is purified by column chromatography over silica gel eluting with hexane/ethyl acetate (70:30) to yield 0.75 g (91%) of the title compound. MS (m/z): 484 (M+1).

### Example 50: [4-[(2-Chloro-4,4-difluoro-spiro[5H-thieno[2,3-c]pyran-7,4'-piperidine]-1'-yl)methyl]-1-(2,6-difluorophenyl)pyrazol-3-yl]methanol

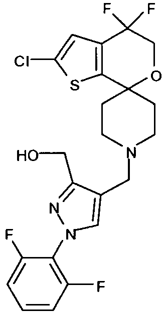

The title compound is prepared essentially as described in Example 49 using 2'-chloro-4',4'-difluoro-4',5'-dihydrospiro[piperidine-4,7'-thieno[2,3-c]pyran] hydrochloride and ethyl 1-(2,6-difluorophenyl)-4-formyl-pyrazole-3-carboxylate.The title compound is obtained in 26% yield. MS (m/z): 502 (M+1).

### Example 51: 2-Chloro-4,4-difluoro-1'-[[1-(2-fluorophenyl)-3-(methoxymethyl)pyrazol-4-yl]methyl]spiro[5H-thieno[2,3-c]pyran-7,4'-piperidine] (L)-Tartrate

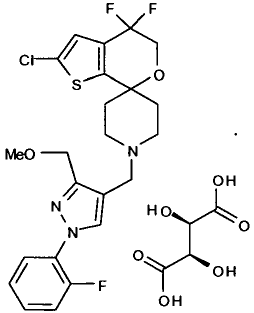

To a solution of 2'-chloro-4',4'-difluoro-4',5'-dihydrospiro[piperidine-4,7'-thieno[2,3-c]pyran] hydrochloride (0.32 g, 1.0 mmol) and 1-(2-fluorophenyl)-3-(methoxymethyl)pyrazole-4-carbaldehyde (0.28 g, 1.2 mmol) in 1,2-dichloroethane (10 mL) is added *N*-methyl morpholine (3.0 equiv, 3.0 mmol). The reaction mixture is stirred at room temperature for 10 minutes and then sodium triacetoxyborohydride (0.53 g, 2.5 mmol) is added and stirred at room temperature for 24 hours. After completion, the reaction mixture was partitioned between dichloromethane (15 mL) and water (15 mL). The aqueous phase is extracted with dichloromethane (3 x 15 mL) and the combined organic extracts are dried over sodium sulfate and concentrated *in vacuo*. The crude mixture is purified by column chromatography over silica gel eluting with dichloromethane/methanol (99:1) to yield 0.27 g (54%) of 2-chloro-4,4-difluoro-1'-[[1-(2-fluorophenyl)-3-(methoxymethyl)-pyrazol-4-yl]methyl]-spiro[5H-thieno[2,3-c]pyran-7,4'-piperidine]. MS (m/z): 498 (M+1).

The tartrate salt is essentially prepared as described in Example 1. MS (m/z): 498 (M+1).

### Receptor occupancy tracer compound: 2-[(2-Fluorophenyl)methyl]-3-(2-fluorospiro[4,5-dihydrothieno[2,3-c]pyran-7,4'-piperidine]-1'-yl)-N,N-dimethyl-propanamide (L)-Tartrate

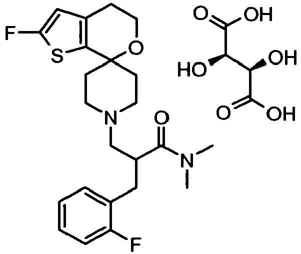

### 1. tert-Butyl 3-(2-fluorospiro[4,5-dihydrothieno[2,3-c]pyran-7,4'-piperidine]-1'-yl) propanoate

2-Fluorospiro[4,5-dihydrothieno[2,2-c]pyran-7,4'-piperidine] (2.7 g, 11.9 mmol) is dissolved in MeOH (60 mL). Then, triethylamine (2.65 mL) and tert-butyl acrylate (3.55 mL, 23.76 mmol) is added and the mixture is heated to 65°C for 5 hr. Heat is removed and reaction mixture is stirred at room temperature overnight. Solvent is evaporated and crude is purified by normal phase Isco chromatography using ethyl acetate/hexane 1/1 as eluent to yield 4.2 g of desired compound as colorless oil. MS (m/z): 356 (M+1).

### 2. tert-Butyl 2-[(2-fluorophenyl)methyl]-3-(2-fluorospiro[4,5-dihydrothieno[2,3-c]pyran-7,4'-piperidine]-1'-yl)propanoate

To a stirred solution of tert-butyl 3-(2-fluorospiro[4,5-dihydrothieno[2,3-c]pyran-7,4'-piperidine]-1'-yl) propanoate (4.9 g, 13.78 mmol) in tetrahydrofuran (41 mL) under N₂ and cooled to -78°C, lithium bis(trimethylsilyl)amide 1M (41,35 mL, 41.45 mmol) is added dropwise. The resulting mixture is stirred at that temperature for 3 hours. Then, 1,3-dimethyl-3,4,5,6-tetrahydro-2(1H)-pyrimidinone (1.33 mL, 11.03 mmol) is added, and the resulting solution is stirred at the same temperature for 30 min. To the resulting mixture, 2-fluorobenzyl bromide (2.33 mL, 19.3 mmol) in dry tetrahydrofuran (1 mL) is added and stirring is continued. The temperature is allowed to go from -78°C to room temperature overnight. The crude reaction mixture is quenched with aqueous saturated solution of ammonium chloride and extracted with ethyl acetate. The organic layer is decanted, dried over magnesium sulfate, the solvent is evaporated and the crude obtained is purified by normal phase Isco chromatography using ethyl acetate/hexane from 5/95 to 20/80 to yield 5.06 g of the title compound obtained as colorless oil. MS (m/z): 464 (M+1).

### 3. 2-[(2-Fluorophenyl)methyl]-3-(2-fluorospiro[4,5-dihydrothieno[2,3-c]pyran-7,4'-piperidine]-1'-yl)propanoic acid trifluoroacetic salt

A mixture of tert-butyl 2-[(2-fluorophenyl)methyl]-3-(2-fluorospiro[4,5-dihydrothieno[2,3-c]pyran-7,4'-piperidine]-1'-yl)propanoate (5.06 g, 10.91 mmol) and trifluoroacetic acid (26.20 mL, 218 mmol) is stirred at room temperature overnight. The solvent is evaporated to dryness and the crude is used without further purification.

### 4. 2-[(2-Fluorophenyl)methyl]-3-(2-fluorospiro[4,5-dihydrothieno[2,3-c]pyran-7,4'-piperidine]-1'-yl)-N,N-dimethyl-propanamide

2-[(2-Fluorophenyl)methyl]-3-(2-fluorospiro[4,5-dihydrothieno[2,3-c]pyran-7,4'-piperidine]-1'-yl)propanoic acid trifluoroacetic salt (5.68 g, 10.89 mmol) is dissolved in dichloromethane (218 mL), then triethylamine (12.14 mL, 87.13 mmol), dimethylamine hydrochloride (1.80 g, 21.78 mmol), 1-(3-dimethylaminopropyl)-3-ethylcarbodiimide Hydrochloride (4.18 g, 21.78 mmol), and 1-hydroxybenzotriazole hydrate (3.34 g, 21.78 mmol) are subsequently added to the solution at 0°C. The mixture is stirred at room temperature for 5 hours. The reaction mixture is treated with aqueous saturated solution of sodium bicarbonate and extracted with dichloromethane (3 x 20 mL). The combined organic layers are dried over magnesium sulfate and the solvent evaporated under reduced pressure. The crude is purified by normal phase Isco chromatography using dichloromethane/2N ammonium in methanol from 100/0 to 90/10 as eluent to give 4.0 g (84.5%) of the title compound. MS (m/z): 435 (M+1).

### 5. 2-[(2-Fluorophenyl)methyl]-3-(2-fluorospiro[4,5-dihydrothieno[2,3-c]pyran-7,4'-piperidine]-1'-yl)-N,N-dimethyl-propanamide (L)-Tartrate

Enantiomeric resolution of racemic 2-[(2-fluorophenyl)methyl]-3-(2-fluorospiro[4,5-dihydrothieno[2,3-c]pyran-7,4'-piperidine]-1'-yl)-*N,N*-dimethyl-propanamide (2.3 g, 5.29 mmol) )is carried out with a Chiralpak AD column using hexane/0.2% dimethyl ethyl amine in ethanol 9/1. The desired compound is obtained in 36% yield as the first eluting enantiomer.

The tartrate salt is essentially prepared as described in example 1. MS (m/z): 435 (M+1).

Literature data (Przydzial and Heisler, 2008, *supra*; Reinscheid, 2006, *supra*) and data generated in nonclinical animal studies support a role for nociceptin antagonists in the treatment of depression, obesity and eating disorders, and migraine. Specifically it is found that nociceptin receptor antagonists are effective in rodent models of depression both alone and in combination with tricyclic or selective serotonergic reuptake inhibitor (SSRI) antidepressants, in rodent models of inhibiting hyperphagia, of inhibiting weight regain following previous weight loss, and in models for migraine. Moreover, studies conducted in nociceptin receptor knockout mice have demonstrated that the action of nociceptin antagonists in the forced swim test (measure of antidepressant activity) and in fasting-induced feeding (anti-obesity activity) is genotype dependent, supporting a specific mechanism of nociceptin antagonist action in these animal models. As the previously described disorders represent common co-morbid clinical conditions a nociceptin receptor antagonist may be particularly effective in these specific patient populations, such as patients with major depressive disorder, binge-eating disorder, overweight, obesity, and obesity with co-morbid clinical mood disorders.

To further demonstrate the characteristics of the present compounds, representative compounds are run in the following in vitro and in vivo assays:

### In Vitro Receptor Binding

Radioligand binding assays are commonly used to determine the affinity (Kᵢ) or potency of a compound to bind to a particular receptor or target protein. A filtration-based [3H]-OFQ/nociceptin receptor binding assay is developed based on previous assay formats (Ardati A, Henningsen RA, Higelin J, Reinscheid RK, Civelli O, Monsma FJ Jr. Mol Pharmacol. 1997 May;51(5):816-24.) with minor modifications. [3H]-OFQ/nociceptin binding assays are carried out in deep-well 96-well plates. [³H]OFQ (final assay concentration 0.2 nM) competition studies are carried out with 5-10 µg of membrane protein (isolated from Chinese hamster ovary cells (CHO cells) expressing cloned human ORL1 receptors) in a final assay volume of 0.5 mL of buffer containing 20 mM HEPES, pH 7.4, 5 mM MgCl₂, 1 mM EGTA, 100 mM NaCl, 0.1% bovine serum albumin. Samples are incubated for 60 min. at room temperature, which is found to be optimal for competition assays. The assays are terminated by filtration through glass fiber filters (Wallac filtermat A) [pretreated with 0.3% polyethylenimine (Sigma) for 1 hr] on a Tometc cell harvester, and the filters are washed three times with 5 mL of ice-cold 50 mM Tris·HCl, pH 7.4. Filtermats are then dried and imbedded with Meltilex scintillant A and the radioactivity counted in a Wallac Microbeta scintillation counter. Specific binding is determined by displacement with 100 nM unlabeled nociceptin. Curves are plotted as the percent of specific binding and IC₅₀ values are determined using a sigmoidal dose response curve with variable slope. Kᵢ values are calculated from the IC₅₀ by the equation of Cheng and Prusoff (Cheng, Y.C., and Prusoff, W.H., Biochem. Pharmacol. 22, 3099-3108 (1973)) where Kᵢ =IC₅₀ x (1+D x K_{d}⁻¹)⁻¹.

Similarly, Kᵢ for the mu, kappa and delta opioids, serotonin, adrenergic, and muscarinic receptors may be determined using membranes expressing the desired receptor and appropriate corresponding radioligand competitor molecules.

Exemplified compounds are tested essentially as described above and are found to have high affinity for the ORL-1 receptor. Kᵢ 's for the ORL-1 receptor for the exemplified compounds are found to be less than 0.9 nM, while the Kᵢ for mu, kappa and delta opioids, serotonin, and dopamine receptors are found to be significantly greater. The compounds of Examples 2, 40, 47, and 50 are tested essentially as described above and are found to have affinities as shown in Table 1, below.

**Table 1. Binding Selectivity**

| Kᵢ (nM) | Example 2 | Example 40 | Example 47 | Example 50 |
|---|---|---|---|---|
| ORL-1 | 0.17 | 0.25 | 0.26 | 0.89 |
| Mu Opioid | >467 | >451 | ND | ND |
| Kappa Opioid | >458 | >430 | ND | ND |
| Delta Opioid | >481 | >479 | ND | ND |
| 5-HT_{1A} | >3180 | ND | ND | ND |
| 5-HT_{1B} | >3580 | ND | ND | >3580 |
| 5-HT_{1D} | >7890 | ND | ND | >8550 |
| 5-HT_{1E} | >5370 | ND | ND | >5370 |
| 5-HT_{1F} | ND | ND | ND | >8520 |
| 5-HT_{2A} | >7180 | ND | ND | >5000 |
| 5-HT_{2B} | 1160 | ND | ND | 42.9 |
| 5-HT_{2C} | >8360 | ND | ND | 4750 |
| 5-HT₄ | >1970 | ND | ND | >4120 |
| 5-HT₅ | >9020 | ND | ND | >9090 |
| 5-HT₆ | >5830 | ND | ND | >5830 |
| 5-HT₇ | >3980 | ND | ND | >3980 |
| Adenosine A3 | 2300 | ND | ND | ND |
| Muscarinic M1 | 1380 | ND | ND | ND |
| Muscarinic M2 | >8000 | ND | ND | ND |
| Muscarinic M3 | >24200 | ND | ND | ND |
| Muscarinic M4 | 6360 | ND | ND | ND |
| Muscarinic M5 | 2800 | ND | ND | ND |

| | | | | |
|---|---|---|---|---|
| ND = not determined | | | | |

Therefore, physiologically relevant doses of the compounds of the invention are not expected to substantially interact with these sites in vivo, and thus are expected to avoid undesired effects associated with such activity.

### In Vitro Functional Blockade of Agonist-Mediated G-protein Activation -GTPγ-[35S] Binding.

Agonist-mediated stimulation of G-protein coupled receptors results in the activation of membrane associated Gαβγ-protein heterotrimer complexes, and represents the first step in the transduction of extracellular signals to modification of intracellular pathways. The first step in activation of receptor-mediated activation of Gαβγ-proteins heterotrimer is the exchange of Gα subunit bound guanosine diphoshpate (GDP) for guanosine triphosphate (GTP). The binding of GTP to the Gα subunit causes dissociation of the heterotrimer subunits, Gβ and Gγ, resulting in the modulation of several intracellular signaling cascades. Measurement of receptor-mediated G-protein activation can be measured using the non-hydrolyzable radiolabeled analog of GTP, GTP-γ-[35S]. Utilizing this methodology, antagonist affinity (Kb) is measured in membranes expressing cloned human ORL1/nociceptin receptors using a GTP-γ-[35S] binding assay according to previously described protocols with minor modifications (DeLapp et al., J Pharmacol Exp Ther. 1999 May;289(2):946-55; Ozaki et al., Eur J Pharmacol. 2000 Aug 18;402(1-2):45-53). Assays are conducted in a 200-µl volume with the following buffer composition: 100 mM NaCl, 20 mM HEPES, 5 mM MgCl₂, 1 mM EDTA, 0.1% BSA, 3 µM GDP, 0.5 nM [³⁵S]GTPγS. ORL1 receptor membrane suspension is added at a concentration of 20 µg protein per well and receptor stimulation is achieved using 300 nM nociceptin/OFQ. Wheat germ agglutinin coated SPA beads (Amersham, Arlington Hts., IL) are added at 1 mg per well to detect membrane-bound [³⁵S]GTPγS. Plates are sealed and incubated for 2 hr. at room temperature. Plates are then placed at 4 °C overnight to allow the SPA beads to settle and then counted in a Wallac Microbeta. Specific [³⁵S]GTPγS binding is determined as the difference in CPM observed in the absence and presence of 10 µM unlabeled GTPγS. Data are plotted as the percent of specific [³⁵S]GTPγS bound. Curves are plotted as the percent of specific binding and IC₅₀ values are determined using a sigmoidal dose response curve with variable slope. Antagonist affinity (K_{b}) is estimated according to DeLapp et al., 1999 using a modification of the equation of Cheng and Prusoff (1973) where K_{b} = IC₅₀ x (1+D x EC₅₀⁻¹) ⁻¹.

Exemplified compounds are tested essentially as described above and are found to be potent antagonists of the ORL-1 receptor. K_{b} 's for the ORL-1 receptor are found to be less than 1.3 nM. The compounds of Examples 2, 40, 47and 50 are tested essentially as described above and are found to have K_{b} 's for the ORL-1 receptor of 0.43, 0.30, 0.15 and 0.54 nM, respectively.

### In Vivo Receptor Occupancy

Receptor occupancy (RO) using LC/MS/MS has been established as a way to measure central target engagement of putative ORL-1 antagonist *in vivo*. Nociceptin/ORL1 receptor occupancy (RO) is measured in the hypothalamus, a structure which contains a high density of nociceptin/ORL1 binding sites that are inside the blood brain barrier, using a novel proprietary nociceptin/ORL1 antagonist RO tracer, 2-[(2-fluorophenyl)methyl]-3-(2-fluorospiro[4,5-dihydrothieno[2,3-c]pyran-7,4'-piperidine]-1'-yl)-*N,N*-dimethyl-propanamide, (RO Tracer). These measurements are made without the need for a radiolabeled tracer as previously published for other receptors with modifications (Chernet E, Martin LJ, Li D, Need AB, Barth VN, Rash KS, Phebus LA. Use of LC/MS to assess brain tracer distribution in preclinical, in vivo receptor occupancy studies: dopamine D2, serotonin 2A and NK-1 receptors as examples. Life Sci. 78(4):340-6, 2005.).

A positive correlation has been established between central nociceptin/ORL1 RO and efficacy in the modulation of the feeding behavior and forced swim test in rodents. Central nociceptin/ORL1 RO is measured at 6 or 24 hours following oral administration of the test compound to rats. Male Sprague-Dawley rats (Harlan Sprague-Dawley, Indianapolis, IN) are treated orally with a test compound, or vehicle (20% Captisol, 25 mM phosphate buffer, pH 2.0). At 6 or 24 hours following administration of test compound/vehicle, all animals are administered an intravenous, 3 µg/kg dose of RO Tracer. It is at the time of RO Tracer administration that RO is considered to be measured. Forty minutes after RO Tracer administration, rats are sacrificed by cervical dislocation and the hypothalamus is removed. The level of RO Tracer is measured in each tissue sample.

The centrally active literature reference standard (-)-*cis*-1-methyl-7-[[4-(2,6-dichlorophenyl)piperidin-1-yl]methyl]-6,7,8,9-tetrahydro-5H-benzocyclohepten-5-ol (SB612111, see Magdalena and Heisler, *supra*), a potent nociceptin/ORL1 receptor selective antagonist, is used as a positive control to establish the RO Tracer level associated with 100% nociceptin/ORL1 RO. SB612111 is administered intravenously at a dose of 30 mg/kg 1 hr. prior to RO Tracer (a dose that results in approximately 100% RO of hypothalamic nociceptin/ORL1 receptors).

Hypothalamic samples are homogenized in 4 volumes (w/v) of acetonitrile containing 0.1% formic acid and centrifuged at 14,000 RPM for 16 min. Supernatants are collected and diluted to a final volume of 0.3 mL with sterile water. Measurement of the RO Tracer is carried out using an Agilent model 1200 HPLC (Agilent Technologies, Palo Alto, CA) and an API 4000 mass spectrometer. The chromatographic separation uses a 2.1 X 50 mm C18 column (Agilent part number 971700-907) and a mobile phase consisting of 38% acetonitrile in water with an overall 0.1% formic acid content. Detection of RO Tracer is accomplished by monitoring the precursor to product ion transition with a mass to charge ratio (m/z) of 435 to 239, with levels quantified by comparison to standards prepared in brain tissue homogenates. Zero percent RO is calculated as the level of RO Tracer in the hypothalamus of vehicle-pretreated animals, which represents the sum of nonspecific and specific binding (all receptors available to the tracer). The lower level of RO Tracer in animals pretreated with the very high intravenous dose of SB612111, the positive control group, represents the nonspecific binding and is assigned the value of 100% occupancy (no receptors available to the tracer). The level of RO Tracer found in hypothalamus from the test compound treated group is linearly interpolated between these two points to calculate RO for the test compound.

Exemplified compounds are tested essentially as described above and are found to have high receptor occupancy at the ORL-1 receptor. Receptor occupancies for exemplified compounds are found to be between about 50 and about 115 % for 3 mg/kg dose after 6 hr., or between about 5 and about 108% RO for 3 mg/kg dose after 24 hr. The receptor occupancies for the compounds of Examples 2, 40, 47, and 50 are assayed essentially as described above for 3 mg/kg dose after 6 hr. and are found to have 76, 70, 84, and 81% RO, respectively. As such, it is expected that the compounds of the present invention have favorable bioavailability and penetration into the CNS to the targeted ORL-1 receptors.

### hERG channel activity

Blockade of K⁺-channel conductance in the heart is associated with cardiotoxicity in the form of QT-wave prolongation. The affinity (Kᵢ) of the exemplified nociceptin receptor antagonists for the human ERG (hERG) K⁺ channel is determined in HEK293 cells expressing cloned hERG using the hERG channel antagonist radioligand [3H]astemizole (2 nM final assay concentration) according to well known procedures (see for example Finlayson K, et al. (Eur J Pharmacol. 412(3):203-12, 2001*)* [3H]Astemizole binding assays are performed at the contract research company Cerep (Paris France) according to standard procedures.

Examples 2, 40, and 50 are assayed essentially as described above and are found to have low activity, with Kᵢ of, 2.89, 1.08, and 7.0 µM, respectively. As such, there is a large separation between the concentrations (in vitro Kᵢ & K_{b}) needed to produce antagonist activity at nociceptin/ORL1 receptors and the concentrations needed for hERG K+-channel activity. Therefore, physiologically relevant doses of the compounds of the invention are not expected to substantially interact with hERG sites in vivo, and thus are expected to have no substantial effect on QT prolongation.

### Forced-Swim Test in Mice (mFST)

mFST is an established in vivo assay for antidepressant activity (Li et al. J Pharmacol Exp Ther. 319(1):254-9, 2006*.*). Mice treated with known clinically effective antidepressants (selective serotonin reuptake inhibitors and/or tricyclic antidepressants) exhibit the behavior of reduced time spent immobile after being placed in a water tank, a behavior associated with despair. The mFST was used to evaluate potential antidepressant-like activity of novel nociceptin/ORL1 antagonists according to previously published methods (Li et al. J Pharmacol Exp Ther. 319(1):254-9,2006.). Briefly, male NIH-Swiss mice (Harlan Sprague-Dawley, Indianapolis, IN) weighing between 25-30 g are used. Group housed animals are removed from the vivarium to the testing area in their own cages and allowed to adapt to the new environment for at least 1 hour before testing. Alternatively, male 129S6 wild type and nociceptin/ORL1 receptor knockout mice were used to confirm dependence of the response to compound was nociceptin/ORL 1 receptor dependent. All compounds are prepared in 20% Captisol, 25 mM phosphate buffer, pH 2.0 on the day of use. Mice are placed in a cylinder (diameter: 10 cm; height: 25 cm) filled with 6 cm of water (22-25°C) for 6 min. The duration of immobility during the last 4 min. of the 6 min. period of the test was scored. A mouse is recorded as immobile when floating motionless or making only those movements necessary to keep its head above water.

Representative compounds are tested essentially as described above and are found to significantly reduce immobilization times. The compounds of Examples 2 and 40 are assayed essentially as described above and are found to have ED₆₀'s of 23 and 23nM respectively, with maximum effects of 56% and 50% reductions in immobilization time, respectively. Therefore compounds of the present invention are expected to have antidepressant activity *in vivo.*

Furthermore, the compounds of the present invention can be used in combination with other known antidepressants to produced enhanced efficacy.

Yet further, these effects of reduced immobilization times are not observed when ORL-1 knockout mice, a strain of mice engineered to lack the ORL-1 receptor, are used, showing that the effect is indeed mediated by the ORL-1 receptor. The compounds of the present invention may be tested essentially as described above using wild type mice in one arm of the study and ORL-1 knockout mice in a second arm of the study, and are found to significantly reduce the immobilization time in the wild type mice, but showed no effect in the knockout mice. A norepinepherine reuptake inhibitor antidepressant, imiprimine, is run as a positive control and is found to reduce immobilization times in both the wild type and knockout mice, showing that the behavioral effect mediated by the norepinepherine reuptake mechanism is intact in the knockout mouse strain.

### Blockade of Fasting-Induced Hyperphagia in mice.

Blockade of fasting-induced hyperphagia in rats is an accepted model for hyperphagic eating disorders. (Hollopeter G, Erickson JC, Seeley RJ, Marsh DJ, Palmiter RD. Response of neuropeptide Y-deficient mice to feeding effectors. Regul Pept. 1998 Sep 25;75-76:383-9.) All experiments are performed on naive twelve-week old male wild type and ORL knockout mice maintained on a 129S6 inbred background. Mice are individually housed a minimum of 3 days prior to the onset of testing to eliminate any effects of stress due to the change from group to individual housing. Three mice/genotype are randomly assigned to each treatment group on the test day. Pre-fast body weight measurements are taken, and food is then removed from cages overnight. Mice are fasted for approximately 15 hr. The next morning, the mice are given one of three doses of drug or vehicle via oral gavage 30 min. prior to gaining access to food. Drugs are dissolved in 20% Captisol dissolved in 25 mM phosphate buffer, pH 2.0. Measurements of body weight are taken immediately prior to drug treatment or 24 hr. after access to food is restored. It is worth noting that all mice independent of genotype lose ∼5-10% body weight following overnight fast. Measurements of food intake are recorded 1 hr following access to food, as indicated, by weight of food remaining at 1 hr. It should be noted that food intake measured is during the light phase, a time during which mice are typically at rest and not normally eating. Following initial testing, mice are rested for 1 week with access to unlimited chow. Following the week rest, mice are retested according to the latin-square design shown in Table 1.

**Table 2. A Latin square design is used to determine dose response curves of anorectic action of novel ORL antagonists in wild type and ORL knockout mice.**

| Treatment Period | | | | |
|---|---|---|---|---|
| Treatment Group | A | B | C | D |
| 1 | Vehicle | A | B | C |
| 2 | A | B | Vehicle | C |
| 3 | B | C | A | Vehicle |
| 4 | C | Vehicle | B | A |

Representative compounds are tested essentially as described above and are found to significantly reduce fasting-induced hyperphagia in mice. Examples 1, 2, 40, 47, and 50, are assayed essentially as described above and are found to substantially block fasting-induced hyperphagia. The effect was not observed in ORL-1 knockout mouse strain, demonstrating that the effect is mediated through the ORL-1 receptor. The 5-HT_{2C} agonist, mCPP is used as a positive control and is found to significantly reduce fasting induced hyperphagia equally in both wild type mice and the ORL-1 knockout mouse strain. As such, it is expected that the compounds of the present invention are useful in the treatment of overweight and/or obesity and/or for weight maintenance, as for example the treatment of binge eating.

### Rat Dural Plasma Protein Extravasation (PPE) Model - Oral Dosing Protocol

All test compounds are prepared in a vehicle solution containing 20% Captisol in 25 mM phosphate buffer (pH 2.0). The positive control compound, sumatriptan, is dissolved in saline. Male Sprague-Dawley rats from Harlan Laboratories (250 to 350 g), that have been fasted overnight, are dosed with test compound, sumatriptan or vehicle by oral gavage (2 mL/kg). Fifty min. post-dose the rats are anesthetized with Nembutal (60 mg/kg, ip) and placed in a stereotaxic frame with the incisor bar set at -2.5 mm. Following a mid-line sagittal scalp incision, 2 pairs of bilateral holes were drilled through the skull (3.2 mm posteriorly, 1.8 and 3.8 mm laterally, all coordinates referenced to bregma). Pairs of stainless steel stimulating electrodes (Rhodes Medical Systems Inc), insulated except at the tips, are lowered through the holes in both hemispheres to a depth of 9.2 mm below the dura.

A solution of fluoroscein isothiocyanate (FITC) dye-labeled bovine serum albumin (BSA) (FITC-BSA) (20 mg/kg, iv), is injected into the femoral vein 2 minutes prior to electrical stimulation of the trigeminal ganglion to function as the marker for protein extravasation. Sixty minutes following dosing with test compound or vehicle, the left trigeminal ganglion is electrically stimulated for 5 minutes at a current intensity of 1.0 mA (5 Hz, 5 minutes duration).

Five minutes following stimulation, the rats are killed by exsanguination with 40 mL of saline which also rinses residual FITC/BSA out of the blood vessels. The top of the skull is removed to collect the dural membranes. The membrane samples are removed from both hemispheres, rinsed with water, and spread flat on microscope slides. The slides are dried for 15 minutes on a slide warmer and cover-slipped with a 70% glycerol/water solution.

A fluorescence microscope equipped with a grating monochromator and a spectrophotometer is used to quantify the amount of FITC-BSA dye in each dural sample. The microscope is equipped with a motorized stage interfaced with a personal computer. This facilitates the computer-controlled movement of the stage, with fluorescence measurements at 25 points (500 µm steps) on each dural sample. The extravasation induced by electrical stimulation of the trigeminal ganglion is an ipsilateral effect (that is occurs only on the side of the dura in which the trigeminal ganglion is stimulated). This allowes the other (unstimulated) half of the dura to be used as a control. The extravasation ratio (that is the ratio of the amount of extravasation in the dura from the stimulated side compared to the unstimulated side) is calculated. Animals dosed with vehicle alone or an ineffective dose of the test compound have an extravasation ratio of approximately 2, while totally effective treatments result in a ratio of approximately 1.

Results are expressed as mean values with standard errors of the mean (± SEM). All statistical evaluations are conducting utilizing ANOVA followed by comparison to the control group by Dunnett's Method. Statistical significance is assumed when p<0.05. Statistical analyses are performed using JMP statistical analysis software (SAS Research Institute, version 6.0.2).

Representative compounds of the present invention are tested essentially as described above and are found to effectively block extravasation in a dose dependent manner. As a result, it is expected that the compounds of the present invention are useful in the treatment of migraine.

### Stability toward reactive metabolite formation

Literature precedent suggests a correlation between reactive metabolite formation and clinical toxicities known as idiosyncratic drug reactions (IDRs), although a direct causal effect has not been established. Assuming that reactive metabolites may play a role in clinical IDRs, minimizing the potential for oxidative bioactivation has been proposed as a means of improving the overall safety profile of compounds containing structural features associated with such reactivity. (see Baillie, Thomas A., Approaches to the Assessment of Stable and Chemically Reactive Drug Metabolites in Early Clinical Trials, Chemical Research in Toxicology, vol 22(2) 2009.) To this end, representative compounds of the present invention and related compounds are screened using a rat hepatic microsomal trapping assay, using glutathione as an endogenous nucleophile, to understand the potential for oxidative bioactivation of the thienyl moiety. Of the compounds tested, those wherein R^{2a} and R^{2b} are hydrogen are found to show evidence of glutathione conjugate formation suggestive of oxidation on the thienyl moiety. Of the compounds tested, those wherein R^{2a} and R^{2b} are fluoro are found to not show glutathione conjugate formation. (See Table 3, below.) The lack of glutathione conjugate formation for the gem-difluoro containing molecules suggests that the gem-difluoro substituent reduces the inherent chemical propensity for bioactiviation as tested in the assay.

**Table 3. Glutathione conjugate formation in hepatic microsomal homogenate**

| Compound | Glutathione conjugate formation |
|---|---|
| 3-fluoro-2-[4-[(2-fluorospiro[4,5-dihydrothieno[2,3-c]pyran-7,4'-piperidine]-1'-yl)methyl]-3-methyl-pyrazol-1-yl]benzamide (L) Tartrate | Yes |
| 3-fluoro-2-[4-[(2,3-difluorospiro[4,5-dihydrothieno[2,3-c]pyran-7,4'-piperidine]-1'-yl)methyl]-3-methyl-pyrazol-1-yl]benzamide (L) Tartrate | Yes |
| (Ex. 14) 2-Chloro-1'-[[1-[3-(methoxymethyl)-2-pyridyl]-3-methyl-pyrazol-4-yl]methyl]spiro[4,5-dihydrothieno[2,3-c]pyran-7,4'-piperidine] (L) Tartrate | Yes |
| (Ex. 10) 2,4,4-Trifluoro-1'-[[1-[3-(methoxymethyl)-2-pyridyl]-3-methyl-pyrazol-4-yl]methyl]spiro[5H-thieno[2,3-c]pyran-7,4'-piperidine] (L) Tartrate | No |
| (Ex. 18) 3-fluoro-2-[3-methyl-4-[(2,4,4-trifluorospiro[5H-thieno[2,3-c]pyran-7,4'-piperidine]-1'-yl)methyl]pyrazol-1-yl]benzamide (L) Tartrate | No |

While it is possible to administer compounds employed in the methods of this invention directly without any formulation, the compounds are usually administered in the form of pharmaceutical compositions comprising at least one compound of Formula I, or a pharmaceutically acceptable salt thereof, as an active ingredient and at least one pharmaceutically acceptable carrier, diluent and/or excipient. These compositions can be administered by a variety of routes including oral, intranasal, transdermal, subcutaneous, intravenous, intramuscular, and pulmonary. Such pharmaceutical compositions and processes for preparing them are well known in the art. *See, e.g.,* Remington: The Science and Practice of Pharmacy (University of the Sciences in Philadelphia, ed., 21st ed., Lippincott Williams & Wilkins Co., 2005).

The compositions are preferably formulated in a unit dosage form, each dosage containing from about 0.1 to about 500 mg, more usually about 1.0 to about 200 mg, as for example between about 5 and 50 mg of the active ingredient. The term "unit dosage form" refers to physically discrete units suitable as unitary dosages for human subjects and other mammals, each unit containing a predetermined quantity of active material calculated to produce the desired therapeutic effect, in association with at least one suitable pharmaceutically acceptable carrier, diluent and/or excipient.

The compounds of Formula I are generally effective over a wide dosage range. For example, dosages per day normally fall within the range of about 0.01 to about 50 mg/kg, more usually from about 0.05 to 5.0 mg/kg, and as for example between 0.1 and 1.0 mg/kg of body weight. In some instances dosage levels below the lower limit of the aforesaid range may be more than adequate, while in other cases still larger doses may be employed without causing any harmful side effect, and therefore the above dosage range is not intended to limit the scope of the invention in any way. It will be understood that the amount of the compound actually administered will be determined by a physician, in the light of the relevant circumstances, including the condition to be treated, the chosen route of administration, the actual compound or compounds administered, the age, weight, and response of the individual patient, and the severity of the patient's symptoms.

## Claims

1. A compound of formula: 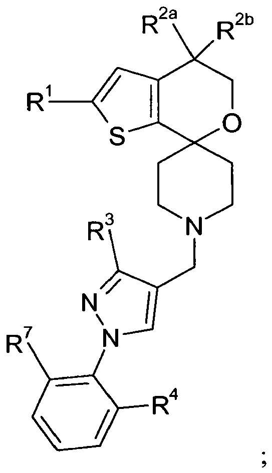 wherein
R¹ is fluoro or chloro;
R^{2a} and R^{2b} are each hydrogen or are each fluoro;
R³ is hydrogen, methyl, hydroxymethyl, or (C₁-C₃) alkoxymethyl;
R⁴ is selected from the group consisting of fluoro, chloro, cyano, cyanomethyl, (C₁-C₃) alkyl, cyclopropyl, hydroxymethyl, methoxy, methoxymethyl, aminocarbonyloxymethyl, methylaminocarbonyloxymethyl, dimethylaminocarbonyloxymethyl, methylcarbonyl, aminocarbonyl, methylaminocarbonyl, dimethylaminocarbonyl, -NR⁵R⁶, -CH₂-NR⁵R⁶, morpholin-4-yl, morpholin-4-ylmethyl, Ar¹, -CH₂Ar¹, 3,3-difluoroazetidin-1-ylmethyl, pyrrolidin-1-ylmethyl, 1-aminocyclopropyl, 1-methylaminocyclopropyl, and 1-dimethylaminocyclopropyl;
R⁵ is hydrogen, C₁-C₄ alkyl, cyclopropyl, hydroxyethyl, methoxyethyl, -C(O)CH₃, or -C(O)O(C₁-C₃) alkyl;
R⁶ is hydrogen or methyl;
R⁷ is hydrogen, fluoro, chloro, methyl, hydroxymethyl, or methoxy; and
Ar¹ is a moiety selected from the group consisting of imidizol-1-yl, imidizol-2-yl, 2-methylimidizol-1-yl, 1-methylimidizol-2-yl, and 1,2,4-triazol-3-yl; or
a pharmaceutically acceptable salt thereof.

2. The compound of Claim 1 wherein R¹ is chloro, or
a pharmaceutically acceptable salt thereof.

3. The compound of either Claim 1 or 2 wherein R^{2a} and R^{2b} are each fluoro, or a pharmaceutically acceptable salt thereof.

4. The compound of Claim 1 wherein R¹ is fluoro and R^{2a} and R^{2b} are each hydrogen, or a pharmaceutically acceptable salt thereof.

5. The compound of any one of Claims 1 through 4 wherein R³ is methyl, or a pharmaceutically acceptable salt thereof.

6. The compound of any one of Claims 1 through 5 wherein R⁴ is fluoro, hydroxymethyl, methoxymethyl, methylcarbonyl or 2-methylimidazol-1-yl, or a pharmaceutically acceptable salt thereof.

7. The compound of any one of Claims 1 through 6 wherein R⁷ is fluoro, or a pharmaceutically acceptable salt thereof.

8. A compound according to Claim 1 selected from the group consisting of 2-chloro-1'-[[1-(2,6-difluorophenyl)-3-methyl-pyrazol-4-yl]methyl]-4,4-difluoro-spiro[5H-thieno[2,3-c]pyran-7,4'-piperidine],
1-(2-(4-((2'-chloro-4',4'-difluoro-4',5'-dihydrospiro[piperidine-4,7'-thieno[2,3-c]pyran]-1-yl)methyl)-3-methyl-1H-pyrazol-1-yl)-3-fluorophenyl)ethanone,
2-chloro-4,4-difluoro-1'-[[1-[2-fluoro-6-(2-methylimidazol-1-yl)phenyl]-3-methyl-pyrazol-4-yl]methyl]spiro[5H-thieno[2,3-c]pyran-7,4'-piperidine], and
[4-[(2-chloro-4,4-difluoro-spiro[5H-thieno[2,3-c]pyran-7,4'-piperidine]-1'-yl)methyl]-1-(2,6-difluorophenyl)pyrazol-3-yl] methanol,
or a pharmaceutically acceptable salt thereof.

9. A pharmaceutical composition comprising a compound according to any one of claims 1 to 8, or a pharmaceutically acceptable salt thereof, and a pharmaceutically acceptable carrier, diluent, or excipient.

10. The pharmaceutical composition of Claim 9 further comprising at least one additional therapeutic ingredient.

11. The pharmaceutical composition of Claim 10 where the additional therapeutic ingredient is an SSRI antidepressant.

12. A compound according to any one of Claims 1 to 8, or a pharmaceutically acceptable salt thereof, for use in therapy.

13. A compound or a pharmaceutically acceptable salt thereof, for use according to claim 12 in the treatment of obesity or overweight in a human.

14. A compound or a pharmaceutically acceptable salt thereof, for use according to claim 12 in the treatment of migraine.

15. A compound or a pharmaceutically acceptable salt thereof, for use according to claim 12 in the treatment of depression.

## Patentansprüche

1. Verbindung der Formel 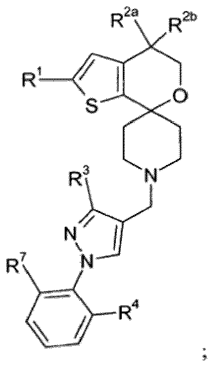 wobei
R¹ für Fluor oder Chlor steht;
R^{2a} und R^{2b} jeweils für Wasserstoff stehen oder jeweils für Fluor stehen;
R³ für Wasserstoff, Methyl, Hydroxymethyl oder (C₁-C₃)-Alkoxymethyl steht;
R⁴ aus der Gruppe ausgewählt ist, die aus Fluor, Chlor, Cyano, Cyanomethyl, (C₁-C₃)-Alkyl, Cyclopropyl, Hydroxymethyl, Methoxy, Methoxymethyl, Aminocarbonyloxymethyl, Methylaminocarbonyloxymethyl, Dimethylaminocarbonyloxymethyl, Methylcarbonyl, Aminocarbonyl, Methylaminocarbonyl, Dimethylaminocarbonyl, -NR⁵R⁶, -CH₂-NR⁵R⁶, Morpholin-4-yl, Morpholin-4-ylmethyl, Ar¹, -CH₂Ar¹, 3,3-Difluorazetidin-1-ylmethyl, Pyrrolidin-1-ylmethyl, 1-Aminocyclopropyl, 1-Methylaminocyclopropyl und 1-Dimethylaminocyclopropyl besteht;
R⁵ für Wasserstoff, (C₁-C₄)-Alkyl, Cyclopropyl, Hydroxyethyl, Methoxyethyl, -C(O)CH₃ oder -C(O)O(C₁-C₃)-Alkyl steht;
R⁶ für Wasserstoff oder Methyl steht;
R⁷ für Wasserstoff, Fluor, Chlor, Methyl, Hydroxymethyl oder Methoxy steht; und
Ar¹ für eine Einheit steht, die aus der Gruppe ausgewählt ist, die aus Imidazol-1-yl, Imidazol-2-yl, 2-Methylimidazol-1-yl, 1-Methylimidazol-2-yl und 1,2,4-Triazol-3-yl besteht;
oder ein pharmazeutisch akzeptables Salz hiervon.

2. Verbindung nach Anspruch 1, wobei R¹ für Chlor steht, oder ein pharmazeutisch akzeptables Salz hiervon.

3. Verbindung nach Anspruch 1 oder 2, wobei R^{2a} und R^{2b} jeweils für Fluor stehen, oder ein pharmazeutisch akzeptables Salz hiervon.

4. Verbindung nach Anspruch 1, wobei R¹ für Fluor steht und R^{2a} und R^{2b} jeweils für Wasserstoff stehen, oder ein pharmazeutisch akzeptables Salz hiervon.

5. Verbindung nach einem der Ansprüche 1 bis 4, wobei R³ für Methyl steht, oder ein pharmazeutisch akzeptables Salz hiervon.

6. Verbindung nach einem der Ansprüche 1 bis 5, wobei R⁴ für Fluor, Hydroxymethyl, Methoxymethyl, Methylcarbonyl oder 2-Methylimidazol-1-yl steht, oder ein pharmazeutisch akzeptables Salz hiervon.

7. Verbindung nach einem der Ansprüche 1 bis 6, wobei R⁷ für Fluor steht, oder ein pharmazeutisch akzeptables Salz hiervon.

8. Verbindung nach Anspruch 1, die aus der Gruppe ausgewählt ist, die aus 2-Chlor-1'-[[1-(2,6-difluorphenyl)-3-methyl-pyrazol-4-yl]methyl]-4,4-difluor-spiro[5H-thieno[2,3-c]pyran-7,4'-piperidin, 1-(2-(4-((2'-Chlor-4',4'-difluor-4',5'-dihydrospiro[piperidin-4,7'-thieno[2,3-c]pyran-1-yl)methyl)-3-methyl-1H-pyrazol-1-yl)-3-fluorphenyl)ethanon, 2-Chlor-4,4'-difluor-1'-[[1-[2-fluor-6-(2-methylimidazol-1-yl)phenyl]-3-methyl-pyrazol-4-yl]methyl]spiro[5H-thieno[2,3-c]pyran-7,4'-piperidin] und [4-[(2-Chlor-4,4-difluor-spiro[5H-thieno[2,3-c]pyran-7,4'-piperidin]-1'-yl]methyl]-1-(2,6-difluorphenyl]pyrazol-3-yl]methanol oder einem pharmazeutisch akzeptablen Salz hiervon besteht.

9. Pharmazeutische Zusammensetzung, die eine Verbindung nach einem der Ansprüche 1 bis 8 oder ein pharmazeutisch akzeptables Salz hiervon und einen pharmazeutisch akzeptablen Träger, ein pharmazeutisch akzeptables Verdünnungsmittel oder einen pharmazeutisch akzeptablen Hilfsstoff umfasst.

10. Pharmazeutische Zusammensetzung nach Anspruch 9, die ferner mindestens einen zusätzlichen therapeutischen Bestandteil umfasst.

11. Pharmazeutische Zusammensetzung nach Anspruch 10, wobei der zusätzliche therapeutische Bestandteil ein SSRI-Antidepressivum ist.

12. Verbindung nach einem der Ansprüche 1 bis 8 oder ein pharmazeutisch akzeptables Salz hiervon zur Verwendung in einer Therapie.

13. Verbindung oder ein pharmazeutisch akzeptables Salz hiervon zur Verwendung gemäß Anspruch 12 bei der Behandlung von Fettleibigkeit oder Übergewicht bei einem Menschen.

14. Verbindung oder ein pharmazeutisch akzeptables Salz hiervon zur Verwendung gemäß Anspruch 12 bei der Behandlung von Migräne.

15. Verbindung oder ein pharmazeutisch akzeptables Salz hiervon zur Verwendung gemäß Anspruch 12 bei der Behandlung von Depression.

## Revendications

1. Composé répondant à la formule suivante : 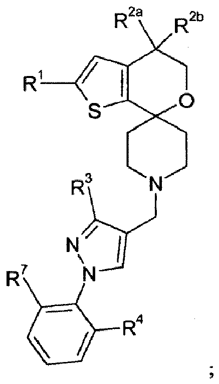 dans laquelle
R¹ représente fluoro ou chloro ;
R^{2a} et R^{2b} représentent chacun hydrogène ou représentent chacun fluoro ;
R³ représente hydrogène, méthyle, hydroxyméthyle, ou alkoxyméthyle en C₁-C₃;
R⁴ est choisi dans le groupe constitué de fluoro, chloro, cyano, cyanométhyle, alkyle en C₁-C₃, cyclopropyle, hydroxyméthyle, méthoxy, méthoxyméthyle, aminocarbonyloxyméthyle, méthylaminocarbonyloxyméthyle, diméthylaminocarbonyloxyméthyle, méthylcarbonyle, aminocarbonyle, méthylaminocarbonyle, diméthylaminocarbonyle, -NR⁵R⁶, -CH₂-NR⁵R⁶, morpholin-4-yle, morpholin-4-ylméthyle, Ar¹, -CH₂Ar¹, 3,3-difluoroazétidin-1-ylméthyle, pyrrolidin-1-ylméthyle, 1-aminocyclopropyle, 1-méthylaminocyclopropyle, et 1-diméthylaminocyclopropyle ;
R⁵ représente hydrogène, alkyle en C₁-C₄, cyclopropyle, hydroxyéthyle, méthoxyéthyle, -C(O)CH₃, ou -C(O)O-alkyle en C₁-C₃ ;
R⁶ représente hydrogène ou méthyle ;
R⁷ représente hydrogène, fluoro, chloro, méthyle, hydroxyméthyle ou méthoxy ; et
Ar¹ est un motif choisi dans le groupe constitué d'imidizol-1-yle, imidizol-2-yle, 2-méthylimidizol-1-yle, 1-méthylimidizol-2-yle, et 1,2,4-triazol-3-yle ; ou un de leurs sels pharmaceutiquement acceptables.

2. Composé selon la revendication 1, R¹ représentant chloro, ou un de ses sels pharmaceutiquement acceptables.

3. Composé selon l'une des revendications 1 ou 2, R^{2a} et R^{2b} représentant chacun fluoro, ou un de ses sels pharmaceutiquement acceptables.

4. Composé selon la revendication 1, R¹ représentant fluoro et R^{2a} et R^{2b} représentant chacun hydrogène, ou un de ses sels pharmaceutiquement acceptables.

5. Composé selon l'une quelconque des revendications 1 à 4, R³ représentant méthyle, ou un de ses sels pharmaceutiquement acceptables.

6. Composé selon l'une quelconque des revendications 1 à 5, R⁴ représentant fluoro, hydroxyméthyle, méthoxyméthyle, méthylcarbonyle ou 2-méthylimidazol-1-yle, ou un de ses sels pharmaceutiquement acceptables.

7. Composé selon l'une quelconque des revendications 1 à 6, R⁷ représentant fluoro, ou un de ses sels pharmaceutiquement acceptables.

8. Composé selon la revendication 1, choisi dans le groupe constitué de
2-chloro-1'-[[1-(2,6-difluorophényl)-3-méthylpyrazol-4-yl]méthyl]-4,4-difluoro-spiro[5H-thiéno[2,3-c]pyran-7,4'-pipéridine],
1-(2-(4-((2'-chloro-4',4'-difluoro-4',5'-dihydrospiro[pipéridine-4,7'-thiéno[2,3-c]pyran]-1-yl)méthyl)-3-méthyl-1 H-pyrazol-1-yl)-3-fluorophényl)éthanone,
2-chloro-4,4-difluoro-1'-[[1-[2-fluoro-6-(2-méthylimidazol-1-yl)phényl]-3-méthylpyrazol-4-yl]méthyl]spiro[5H-thiéno[2,3-c]pyran-7,4'-pipéridine], et
[4-[(2-chloro-4,4-difluoro-spiro[5H-thiéno[2,3-c]pyran-7,4'-pipéridine]-1'-yl)méthyl]-1-(2,6-difluorophényl)pyrazol-3-yl]méthanol,
ou un de leurs sels pharmaceutiquement acceptables.

9. Composition pharmaceutique comprenant un composé selon l'une quelconque des revendications 1 à 8, ou un de ses sels pharmaceutiquement acceptables, et un support, diluant ou excipient pharmaceutiquement acceptable.

10. Composition pharmaceutique selon la revendication 9, comprenant un outre au moins un ingrédient thérapeutique supplémentaire.

11. Composition pharmaceutique selon la revendication 10, ledit ingrédient thérapeutique supplémentaire étant un antidépresseur de type ISRS.

12. Composé selon l'une quelconque des revendications 1 à 8, ou un de ses sels pharmaceutiquement acceptables, pour une utilisation en thérapie.

13. Composé, ou un de ses sels pharmaceutiquement acceptables, pour l'utilisation selon la revendication 12 dans le traitement de l'obésité ou de la surcharge pondérale chez l'homme.

14. Composé, ou un de ses sels pharmaceutiquement acceptables, pour l'utilisation selon la revendication 12 dans le traitement de la migraine.

15. Composé, ou un de ses sels pharmaceutiquement acceptables, pour l'utilisation selon la revendication 12 dans le traitement de la dépression.
